(19) Europäisches Patentamt / European Patent Office / Office européen des brevets

(11) **EP 2 467 381 B1**

(12) # EUROPÄISCHE PATENTSCHRIFT

(45) Veröffentlichungstag und Bekanntmachung des Hinweises auf die Patenterteilung:
**30.04.2014 Patentblatt 2014/18**

(21) Anmeldenummer: **10747439.7**

(22) Anmeldetag: **17.08.2010**

(51) Int Cl.:
***C07D 417/06*** *(2006.01)* ***A61K 31/427*** *(2006.01)*
***A61P 31/18*** *(2006.01)*

(86) Internationale Anmeldenummer:
**PCT/EP2010/061923**

(87) Internationale Veröffentlichungsnummer:
**WO 2011/020822 (24.02.2011 Gazette 2011/08)**

(54) **SUBSTITUIERTE (THIAZOLYL-CARBONYL)IMIDAZOLIDINONE UND IHRE VERWENDUNG ZUR BEHANDLUNG VON RETROVIRALEN KRANKHEITEN**

SUBSTITUTED (THIAZOLYL-CARBONYL)IMIDAZOLIDINONES AND THIER USE FOR THE TREATMENT OF RETROVIRAL DISEASES

(THIAZOLYL-CARBONYL)IMIDAZOLIDINONES SUBSTITUES ET LEUR UTILISATION POUR LE TRAITEMENT DE MALADIES RETROVIRAL

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **17.08.2009 DE 102009038123**

(43) Veröffentlichungstag der Anmeldung:
**27.06.2012 Patentblatt 2012/26**

(73) Patentinhaber: **AiCuris GmbH & Co. KG**
**42117 Wuppertal (DE)**

(72) Erfinder:
- **THEDE, Kai**
**10405 Berlin (DE)**
- **GRESCHAT, Susanne**
**40237 Duesseldorf (DE)**
- **GERICKE, Kersten Matthias**
**42115 Wuppertal (DE)**
- **WILDUM, Steffen**
**58332 Schwelm (DE)**
- **PAULSEN, Daniela**
**42105 Wuppertal (DE)**

(74) Vertreter: **Kilger, Ute et al**
**Boehmert & Boehmert**
**Pettenkoferstrasse 20-22**
**80336 München (DE)**

(56) Entgegenhaltungen:
**WO-A1-2010/075962 WO-A2-02/055079**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 467 381 B1

Printed by Jouve, 75001 PARIS (FR)

## Beschreibung

Die vorliegende Erfindung betrifft neue substituierte (Thiazolyl-carbonyl)imidazolidinone, Verfahren zu ihrer Herstellung, besagte Verbindungen zur Verwendung zur Behandlung und/oder Prophylaxe von Krankheiten sowie ihre Verwendung zur Herstellung von Arzneimitteln zur Behandlung und/oder Prophylaxe von Krankheiten, insbesondere von retroviralen Erkrankungen, bei Menschen und/oder Tieren.

HIV (Virus der humanen Immundefizienz) verursacht eine chronisch-persistente, progrediente Infektion. Die Erkrankung verläuft über verschiedene Stadien von der asymptomatischen Infektion bis zum Krankheitsbild AIDS (*Acquired Immunodeficiency Syndrom*). AIDS ist das finale Stadium der durch Infektion hervorgerufenen Erkrankung. Charakteristisch für die HIV/AIDS-Erkrankung ist die lange klinische Latenzzeit mit persistierender Virämie, die im Endstadium zum Versagen der Immunabwehr führt.

Durch die Einführung der anti-HIV Kombinationstherapie gelang es in den 90-er Jahren, die Krankheitsprogression nachhaltig zu verlangsamen und damit die Lebenserwartung HIV-infizierter Patienten substantiell zu verlängern (Palella et al., N. Engl. J. Med. 1998, 238, 853-860).

Die derzeit auf dem Markt befindlichen anti-HIV-Substanzen hemmen die Replikation des HI-Virus durch Inhibition der essentiellen viralen Enzyme Reverse Transkriptase (RT), Protease oder Integrase oder des Eintritts von HIV in die Zielzelle (Übersicht in Flexner, Nature Reviews Drug Discovery 2007, 6, 959-966). Es existieren zwei Klassen von RT-Inhibitoren: Nukleosidische und nukleotidische RT-Inhibitoren (NRTI) wirken durch kompetitive Inhibition oder Kettenabbruch bei der DNA-Polymerisation. Nicht-nukleosidische RT-Inhibitoren (NNRTI) binden allosterisch an einer hydrophoben Tasche in der Nähe des aktiven Zentrums der RT und vermitteln eine Konformationsänderung des Enzyms. Die derzeit verfügbaren Proteaseinhibitoren (PI) blockieren das aktive Zentrum der viralen Protease und verhindern somit die Reifung neuentstehender Partikel zu infektiösen Virionen. Der einzige derzeit zugelassene Integrase-Inhibitor Raltegravir bindet in das aktive Zentrum der HIV-Integrase und verhindert die Integration der proviralen DNA in das Wirtszellgenom. "Entry-Inhibitoren" (Fusions-Inhibitoren und Korezeptor-Antagonisten) verhindern die HIV-Infektion von Zellen durch Interaktion mit dem HIV-Hüllprotein oder durch Blockierung der zellulären Korezeptoren CCR5 oder CXCR4.

Da die Monotherapie mit den momentan verfügbaren anti-HIV-Medikamenten innerhalb sehr kurzer Zeit zum Therapieversagen durch Selektion resistenter Viren führt, erfolgt üblicherweise eine Kombinationstherapie mit mehreren anti-HIV-Substanzen aus verschiedenen Klassen (*highly active antiretroviral therapy* = HAART; Carpenter et al., J. Am. Med. Assoc. 2000, 283, 381-390).

Trotz der Fortschritte in der antiretroviralen Chemotherapie zeigen neuere Untersuchungen, dass mit den zur Verfügung stehenden Medikamenten eine Eradikation von HIV und damit verbunden eine Heilung der HIV-Infektion nicht zu erwarten ist. Das latente Virus verbleibt in ruhenden Lymphozyten und stellt ein Reservoir für eine Reaktivierung und damit für eine erneute Virusausbreitung dar (Finzi et al., Nature Med. 1999, 5, 512-517; Ramratnam et al., Nature Med. 2000, 6, 82-85). HIVinfizierte Patienten sind daher zeitlebens auf eine effiziente antivirale Therapie angewiesen. Trotz Kombinationstherapie kommt es nach einiger Zeit zur Selektion resistenter Viren. Da für jede therapeutische Klasse charakteristische Resistenzmutationen akkumulieren, bedeutet das Versagen einer Therapie oft einen Wirkverlust der kompletten Substanzklasse. Diese Kreuzresistenzproblematik ist bei der Klasse der NNRTIs am ausgeprägtesten, da hier oft schon eine einzelne Punktmutation in der RT ausreichen kann, um einen Wirkverlust aller NNRTIs zu bewirken (Übersicht in Kavlick & Mitsuya, Antiretroviral Chemotherapy (Hrsg. De Clercq E.), 2001, ASM Press, 279-312).

Begünstigt wird die Entstehung von Resistenzen meist durch die schlechte Compliance der Patienten, die durch ein ungünstiges Nebenwirkungsprofil und kompliziertes Dosierungsschema der anti-HIV-Medikamente hervorgerufen wird.

Somit besteht ein dringender Bedarf nach neuen therapeutischen Optionen zur Bekämpfung der HIV-Infektion. Dazu ist es ein vordringliches Ziel der Therapieforschung zu HIV, neue chemische Leitstrukturen zu identifizieren, die entweder ein neues Target in der Vermehrung des HIV adressieren und/oder gegen die wachsende Zahl resistenter klinischer HIV-Isolate wirksam sind.

EP 377 457 A1 und EP 388 909 A2 beschreiben Thiazolcarboxamide mit antithrombotischer, antiallergischer, antiinflammatorischer Wirkung sowie mit Wirkung als Vasodilatoren und 5-Lipooxigenaseinhibitoren, WO 03/78413 A1, WO 2004/058255 A1 beschreiben Thiophencarboxamide mit Wirkung auf den Canabinoid-CB1-Rezeptor, WO 03/041711 A1 beschreibt Thiophencarboxamide als Orexinrezeptorantagonisten, WO 2005/115389 A2 beschreibt Thiazolcarboxamide zur Behandlung einer negativen Energiebalance in Wiederkäuern, WO 2006/023462 A1 als Histamin-H3-Rezeptorantagonisten, und WO 2006/062984 und WO 2006/062982 A2 als Inhibitoren verschiedener Kinasen. WO 02/055079 beschreibt Aza - Naphthalenyl carboxamide als HIV Integrase Inhibitoren

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen, die die zuvor beschriebenen Nachteile nicht aufweisen.

Überraschenderweise wurde gefunden, dass die in der vorliegenden Erfindung beschriebenen substituierten

(Thiazolyl-carbonyl)imidazolidinone antiviral wirksam sind.

Gegenstand der Erfindung sind Verbindungen der Formel

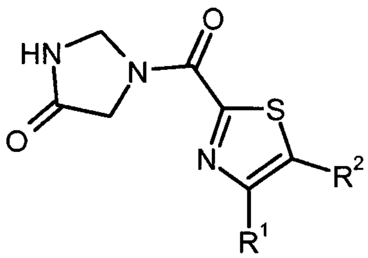

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin
$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können, und

$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin
$(C_1-C_4)$-Alkyl und $(C_1-C_4)$-Alkoxy ihrerseits ein- bis dreifach, gleich oder verschieden, mit Resten substituiert sein können, die ausgewählt werden aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

und ihre Salze, ihre Solvate und die Solvate ihrer Salze.

Erfindungsgemäße Verbindungen sind die Verbindungen der Formeln (I) und (Ia) und deren Salze, Solvate und Solvate der Salze, sowie die von den Formeln (I) und (Ia) umfassten, nachfolgend als Ausführungsbeispiel(e) genannten Verbindungen und deren Salze, Solvate und Solvate der Salze, soweit es sich bei den von den Formeln (I) und (Ia) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb auch die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl sowie die Alkylteile in Alkoxy und Alkoxycarbonyl stehen für gerakettiges oder verzweigtes Alkyl und umfassen, wenn nicht anders angegeben, ($C_1$-$C_6$)-Alkyl, insbesondere ($C_1$-$C_4$)-Alkyl, wie z.B. Methyl, Ethyl, Propyl, Isopropyl, Butyl, Isobutyl.

Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.

Alkoxycarbonyl steht beispielhaft und vorzugsweise für Methoxycarbonyl, Ethoxycarbonyl, n-Propoxycarbonyl, Isopropoxycarbonyl, t-Butoxycarbonyl, n-Pentoxycarbonyl und n-Hexoxycarbonyl.

Heterocyclyl steht für einen monocyclischen, heterocyclischen Rest mit 4 bis 7, vorzugsweise 5 bis 6 Ringatomen und bis zu 3, vorzugsweise bis zu 2 Heteroatomen und/oder Heterogruppen aus der Reihe N, O, S, SO, $SO_2$, wobei ein Stickstoffatom auch ein N-Oxid bilden kann. Der Heterocyclus kann gesättigt oder teilweise ungesättigt sein. Bevorzugt sind 5- bis 7-gliedrige, monocyclische gesättigte Heterocyclen mit bis zu zwei Heteroatomen aus der Reihe O, N und S, beispielhaft und vorzugsweise 1,4-Oxazepanyl, Oxetan-3-yl, Pyrrolidin-1-yl, Pyrrolidin-2-yl, Pyrrolidin-3-yl, Tetrahydrofuranyl, Tetrahydrothienyl, Pyranyl, 1,3-Thiazolidinyl, Piperidin-1-yl, Piperidin-2-yl, Piperidin-3-yl, Piperidin-4-yl, Thiopyranyl, Morpholin-2-yl, Morpholin-3-yl, Morpholin-4-yl, Thiomorpholin-2-yl, Thiomorpholin-3-yl, Thiomorpholin-4-yl, Perhydroazepinyl, Piperazin-1-yl, Piperazin-2-yl.

Halogen steht für Fluor, Chlor, Brom oder Jod, wobei Fluor und Chlor bevorzugt sind, wenn nichts anderes angegeben ist.

Mono-($C_1$-$C_4$)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.

Di-($C_1$-$C_4$)-alkylamino steht im Rahmen der Erfindung für eine Amino-Gruppe mit zwei gleichen oder verschiedenen geradkettigen oder verzweigten Akylsubstituenten, die jeweils 1 bis 4 Kohlenstoffatome aufweisen. Beispielhaft und vorzugsweise seien genannt: *N*,*N*-Dimethylamino, *N*,*N*-Diethylamino, *N*-Ethyl-*N*-methylamino, *N*-Methyl-*N*-*n*-propylamino, *N*-Isopropyl-*N*-*n*-propylamino, *N*,*N*-Diisopropylamino, *N*-n-Butyl-*N*-methylamino, *N*-*tert*.-Butyl-*N*-methylamino, *N*-Methyl-*N*-*n*-pentylamino und *N*-*n*-Hexyl-*N*-methylamino.

($C_3$-$C_7$)-Cycloalkyl steht im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Die oben aufgeführten allgemeinen oder in Vorzugsbereichen angegebenen Restedefinitionen gelten sowohl für die Endprodukte der Formeln (I) und (Ia) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,
worin
$(C_1-C_4)$-Alkoxy seinerseits ein- bis dreifach, gleich oder verschieden, mit Resten substituiert sein kann, die ausgewählt werden aus der Reihe Halogen, Cyano, Hydroxy, $(C_1-C_4)$-Alkoxy, Amino, Mono-$(C_1-C_4)$-alkyl-amino, Di-$(C_1-C_4)$-alkylamino, $(C_3-C_7)$-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Trifluormethoxy, Methyl und $(C_1-C_3)$-Alkoxy,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano und Trifluormethyl,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano, und

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano

und ihre Salze, ihre Solvate und die Solvate ihrer Salze.

Gegenstand der Erfindung sind auch Verbindungen der Formel

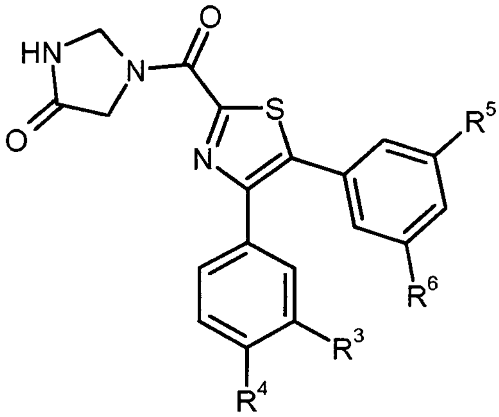

(Ia),

in welcher

$R^3$ für Halogen oder Cyano steht,
$R^4$ für Wasserstoff oder Halogen steht,
$R^5$ für Halogen, Cyano oder Trifluormethyl steht, und
$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Halogen oder Cyano steht,
$R^4$ für Wasserstoff oder Halogen steht,
$R^5$ für Halogen oder Cyano steht, und
$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Fluor, Chlor oder Cyano steht,
$R^4$ für Wasserstoff, Chlor oder Fluor steht,
$R^5$ für Fluor, Chlor oder Cyano steht, und
$R^6$ für Wasserstoff, Chlor oder Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Wasserstoff oder Fluor steht,
$R^5$ für Halogen oder Cyano steht, und
$R^6$ für Wasserstoff oder Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Fluor steht,
$R^5$ für Chlor oder Cyano steht, und
$R^6$ für Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,

$R^4$ für Fluor steht,
$R^5$ für Chlor oder Cyano steht, und
$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Wasserstoff steht,
$R^5$ für Chlor oder Cyano steht, und
$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Wasserstoff steht,
$R^5$ für Chlor oder Cyano steht, und
$R^6$ für Fluor steht,

und ihre Salze, ihre Solvate und die Solvate ihrer Salze.

Gegenstand der Erfindung ist weiterhin ein Verfahren zur Herstellung der Verbindungen der Formeln (I) und (Ia), wobei Verbindungen der Formel

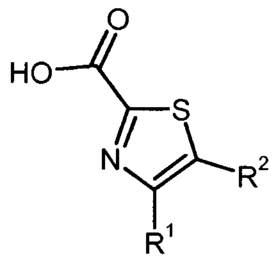

(II),

in welcher
$R^1$ und $R^2$ die oben angegebene Bedeutung aufweisen,
mit Imidazolidin-4-on oder einem Salz von Imidazolidin-4-on umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoffe wie Benzol oder Toluol, Nitromethan, Tetrahydrofuran, 1,4-Dioxan, Dimethylformamid oder Acetonitril. Ebenso ist es möglich, Gemische der Lösemittel einzusetzen. Besonders bevorzugt sind Dichlormethan, Dimethylformamid, Tetrahydrofuran oder Toluol.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N*,*N'*-Diethyl-, *N*,*N'*-Dipropyl-, *N*,*N'*-Diisopropyl-, *N*,*N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-di-hydrochinolin, oder Propanphosphonsäureanhydrid, oder Isobutylchloroformat, oder Bis-(2-oxo-3-oxazolidinyl)-phosphorylchlorid, oder *O*-(Benzotriazol-1-yl)-*N*,*N*,*N'*,*N'*-tetramethyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzo-triazol-1-yl)-*N*,*N*,*N'*,*N'*-tetramethyluroniumhexafluorophosphat (HATU), oder 1-Hydroxybenztriazol (HOBt), oder Benzotriazol-1-yloxytris(dimethylamino)phosphoniumhexafluorophosphat (BOP), oder Benzotriazol-1-yloxytris(pyrrolidino)phospho-niumhexafluorophosphat (PyBOP), oder *N*-Hydroxysuccinimid, oder Mischungen aus diesen, mit Basen.

Vorzugsweise wird die Kondensation mit PyBOP, TBTU oder mit EDC in Gegenwart von HOBt durchgeführt.

In einem alternativen Verfahren können die Verbindungen der Formel (II) zunächst mit Thionylchlorid und in der zweiten Stufe mit Verbindungen der Formel $R^3$ oder einem Salz von Verbindungen der Formel $R^3$ in Gegenwart einer Base, wie z. B. Triethylamin, umgesetzt werden.

Die nach den oben angegebenen Verfahren hergestellten Verbindungen der Formel (I) und (Ia) tragen gegebenenfalls Schutzgruppen, die nach dem Fachmann bekannten Bedingungen abgespalten werden können, um weitere Verbindungen der Formel (I) und (Ia) zu erhalten.

Die Verbindungen der Formel (II) sind bekannt oder können hergestellt werden, indem Verbindungen der Formel

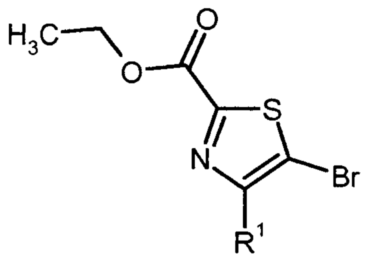

(III),

in welcher

$R^1$ die oben angegebene Bedeutung aufweist,

unter Suzuki-Kupplungsbedingungen mit Verbindungen der Formel

$R^2$-Q (IV),

in welcher

$R^2$ die oben angegebene Bedeutung aufweist und

Q für $-B(OH)_2$, einen Boronsäure-Ester, bevorzugt Boronsäurepinakolester, oder $-BF3^-K^+$ steht,

umgesetzt werden.

Die Suzuki-Kupplungen erfolgen im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis 130°C bei Normaldruck.

Katalysatoren sind beispielsweise für Suzuki-Reaktionsbedingungen übliche Palladium-Katalysatoren, bevorzugt sind Katalysatoren wie z.B. Dichlorbis(triphenylphosphin)palladium, Tetrakistriphenylphosphinpalladium(0), Palladium(II)acetat, Palladium(II)acetat/Triscyclohexylphosphin oder Bis(diphenylphosphanferroce-nyl)palladium(II)chlorid oder Palladium(II)acetat mit einem Liganden wie Dicyclohexyl[2',4',6'-tri(propan-2-yl)biphenyl-2-yl]phosphan.

Zusatzreagenzien sind beispielsweise Kaliumacetat, Cäsium-, Kalium- oder Natriumcarbonat, Kalium-tert.-butylat, Cäsiumfluorid oder Kaliumphosphat, bevorzugt sind Zusatzreagenzien wie z.B. Kaliumacetat und/oder eine wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon, oder Gemische der Lösungsmittel mit Alkoholen wie Methanol oder Ethanol und/oder Wasser, bevorzugt ist 1,2-Dimethoxyethan.

In einem alternativen Verfahren können die Verbindungen der Formel (III) zunächst mit einer Verbindung der Formel (IV) so unter Suzuki-Kupplungsbedingungen umgesetzt werden, dass der entsprechende Ester isoliert werden kann. Dann kann in einem zweiten Schritt dieser Ester durch Verseifung mit einer Base in eine Verbindung der Formel (II) überführt werden.

Die Verseifung des Esters mit einer Base erfolgt im Allgemeinen in inerten Lösungsmitteln, bevorzugt in einem Temperaturbereich von Raumtemperatur bis zum Rückfluss des Lösungsmittels bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt sind Lithium-, Kalium- oder Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert-butylether, 1,2-Dimethoxyethan, 1,4-Dioxan, Tetrahydrofuran, Glykoldimethylether oder Diethylenglykoldimethylether, Alkohole wie Methanol, Ethanol, n-Propanol, iso-Propanol, n-Butanol oder tert-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Wasser, oder Gemische von Lösungsmitteln. Als Lösungsmittel sind 1,4-Dioxan, Tetrahydrofuran und/oder Methanol bevorzugt. Bevorzugt ist Lithiumhydroxid in Tetrahydrofuran- oder 1,4-Dioxan-Wasser-Gemischen oder Kaliumhydroxid in Methanol.

Die Verbindungen der Formel (IV) sind bekannt oder lassen sich nach bekannten Verfahren aus den entsprechenden Edukten synthetisieren.

Die Verbindungen der Formel (III) sind bekannt oder können hergestellt werden, indem Verbindungen der Formel

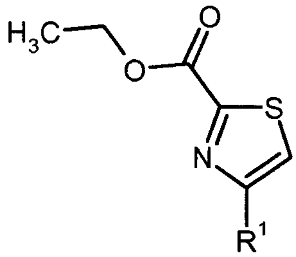

(V),

in welcher

$R^1$ die oben angegebene Bedeutung aufweist, bromiert werden.

Inerte Lösungsmittel für die Bromierung sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Hexan oder Cyclohexan, organische Carbonsäuren wie Essigsäure oder andere Lösungsmittel wie Ethylacetat, Dimethylformamid oder Dimethylsulfoxid. Ebenso ist es möglich, Gemische der genannten Lösungsmittel einzusetzen. Bevorzugt sind Essigsäure, Diethylether, Dioxan, Tetrahydrofuran, Ethylacetat, Trichlormethan und/oder Tetrachlormethan.

Als Bromierungsmittel eignen sich die üblichen anorganischen oder organischen Reagenzien. Hierzu gehören bevorzugt Brom, *N*-Bromsuccinimid, Kupferdibromid, Pyridinhydrotribromid, Dimethylbenzylammoniumtribromid oder Phenyltrimethylammoniumtribromid. Besonders bevorzugt sind N-Bromsuccinimid, Brom und Kupferdibromid.

Die Bromierung wird im Allgemeinen in einem Temperaturbereich von -20°C bis +150°C, bevorzugt von 0°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die Verbindungen der Formel (V) sind bekannt oder können hergestellt werden, indem Verbindungen der Formel

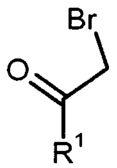

(VI),

in welcher

$R^1$ die oben angegebene Bedeutung aufweist, mit Ethylamino(thioxo)acetat umgesetzt werden.

Inerte Lösungsmittel für die diesen Schritt sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Hexan oder Cyclohexan, Alkohole wie Methanol oder Ethanol oder andere Lösungsmittel wie Ethylacetat, Dimethylformamid oder Dimethylsulfoxid. Ebenso ist es möglich, Gemische der genannten Lösungsmittel einzusetzen. Bevorzugt ist Ethanol.

Die Umsetzung wird im Allgemeinen in einem Temperaturbereich von -20°C bis +150°C, bevorzugt von 0°C bis +100°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die Verbindungen der Formel (VI) sind bekannt oder lassen sich nach bekannten Verfahren aus den entsprechenden Edukten synthetisieren.

Gegenstand der Erfindung ist weiterhin ein Verfahren zur Herstellung der Verbindungen der Formeln (I) und (Ia), wobei Verbindungen der Formel

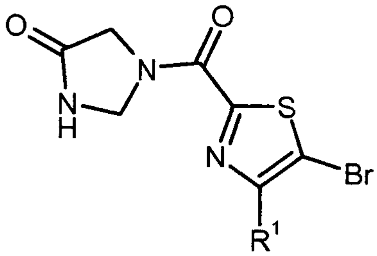

(VII),

in welcher
$R^1$ die oben angegebene Bedeutung aufweist,
unter den oben angegebenen Suzuki-Kupplungsbedingungen mit Verbindungen der Formel

$R^2$-Q (IV),

in welcher
$R^2$ die oben angegebene Bedeutung aufweist und

Q für $-B(OH)_2$, einen Boronsäure-Ester, bevorzugt Boronsäurepinakolester, oder $-BF_3^-K^+$ steht,

umgesetzt werden.

Die Verbindungen der Formel (VII) sind bekannt oder können hergestellt werden, indem Verbindungen der Formel

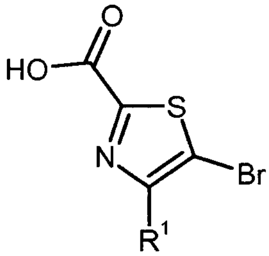

(VIII),

in welcher
$R^1$ die oben angegebene Bedeutung aufweist,
mit Imidazolidin-4-on oder einem Salz von Imidazolidin-4-on entsprechend der oben angegebenen Reaktion von (II) zu (I) bzw. (Ia), umgesetzt werden.

Die Verbindungen der Formel (VIII) sind bekannt oder können hergestellt werden, indem in Verbindungen der Formel

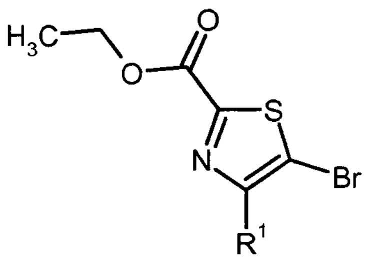

(III),

in welcher
$R^1$ die oben angegebene Bedeutung aufweist,
der Ester mit einer Base, wie oben bei dem alternativen Verfahren von (III) zu (II) beschrieben, verseift wird.

Die Herstellung der erfindungsgemäßen Verbindungen kann durch das folgende Syntheseschema verdeutlicht werden.

Syntheseschema:

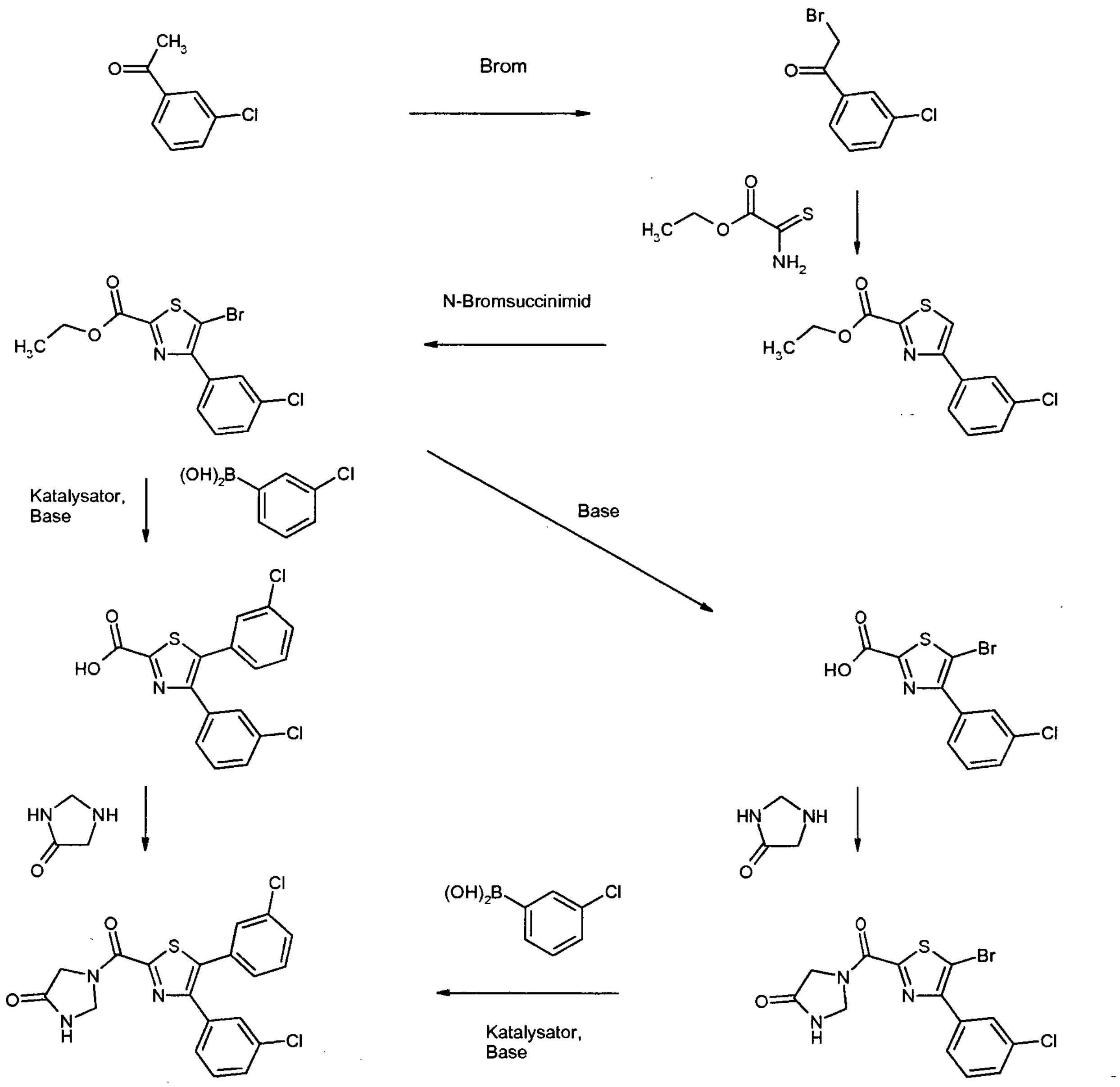


Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die Verbindungen der vorliegenden Erfindung zeichnen sich insbesondere durch ein vorteilhaftes anti-retrovirales Wirkspektrum aus.

Weiterer Gegenstand der vorliegenden Erfindung ist der Einsatz der erfindungsgemäßen Verbindungen zur Behandlung und/oder Prophylaxe von Erkrankungen, die durch Retroviren hervorgerufen werden, insbesondere von HI-Viren.

Weiterer Gegenstand der vorliegenden Erfindung ist erfindungsgemäße Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist erfindungsgemäße Verbindungen zur Verwendung in

einem Verfahren zur Behandlung und/oder Prophylaxe von Erkrankungen, insbesondere der zuvor genannten Erkrankungen, unter Verwendung einer therapeutisch wirksamen Menge der erfindungsgemäßen Verbindungen.

Als Indikationsgebiete in der Humanmedizin können beispielsweise genannt werden:

1.) Die Behandlung und Prophylaxe von menschlichen Retrovirusinfektionen

2.) Die Behandlung und Prophylaxe von durch HIV-1 (Virus der humanen Immundefizienz; früher HTLV III/LAV genannt) und HIV-2 verursachten Infektionen und Erkrankungen (AIDS) und den damit assoziierten Stadien wie ARC (AIDS related complex) und LAS (Lymphadenopathie-Syndrom) sowie der durch dieses Virus verursachten Immunschwäche und Enzephalopathie.

3.) Die Behandlung von HIV-Infektionen hervorgerufen durch einfach-, mehrfach- oder multi-resistente HI-Viren. Resistente HI-Viren bedeutet z.B. Viren mit Resistenzen gegen nukleosidische RT-Inhibitoren (NRTI), nicht-nukleosidische RT-Inhibitoren (NNRTI) oder Proteaseinhibitoren (PI) oder Viren mit Resistenzen gegen andere Wirkprinzipien, z.B. T20 (Fusionsinhibitoren).

4.) Für die Behandlung oder die Prophylaxe des AIDS-carrier Zustandes (AIDS-Überträger-Zustand).

5.) Für die Behandlung oder die Prophylaxe einer HTLV-I oder HTLV-II Infektion

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Insbesondere geeignet sind die Substanzen zur Bekämpfung von HI-Viren, die Resistenzen gegen bekannte nicht-nukleosidische Inhibitoren der Reversen Transkripatse, wie z.B. Efavirenz oder Nevirapin, zeigen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können auch vorteilhaft, insbesondere in den oben aufgeführten Punkten 2, 3 und 4, als Bestandteile einer Kombinationstherapie mit einer oder mehreren anderen, in diesen Anwendungsbereichen aktiven Verbindungen eingesetzt werden. Beispielhaft können diese Verbindungen in Kombination mit wirksamen Dosen von antiviral wirksamen Substanzen, die auf den unten aufgeführten Wirkprinzipien beruhen, eingesetzt werden:

Inhibitoren der HIV Protease; beispielhaft seien genannt: Saquinavir, Indinavir, Ritonavir, Nelfinavir, Amprenavir, Lopinavir, Atazanavir, Fosamprenavir, Tipranavir, Darunavir;

Nukleosidische, nukleotidische und nicht-nukleosidische Inhibitoren der HIV Reversen Transkriptase; beispielhaft seien genannt: Zidovudin, Lamivudin, Didanosin, Zalcitabin, Stavudin, Lamivudin, Abacavir, Tenofovir, Adefovir, Emtricitabin, Amdoxovir, Apricitabin, Racivir, Nevirapin, Delavirdin, Efavirenz, Etravirin, Rilpivirin, UK-453,061;

Inhibitoren der HIV Integrase, beispielhaft seien genannt: Raltegravir, Elvitegravir;

Inhibitoren der HIV Fusion; beispielhaft sei genannt: Enfuvirtid;

Inhibitoren der CXCR4/CCR5/gp120 Wechselwirkung; beispielhaft seien genannt: Maraviroc, Vicriviroc, INCB009471, AMD-070;

Inhibitoren der Polyproteinreifung; beispielhaft sei genannt: Bevirimat.

Diese Auswahl soll zur Verdeutlichung der Kombinationsmöglichkeiten, nicht jedoch zur Einschränkung auf die hier aufgeführten Beispiele dienen; prinzipiell ist jede Kombination der erfindungsgemäßen Verbindungen mit antiviral wirksamen Substanzen als im Rahmen der Erfindung zu betrachten.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human- als auch in der Veterinärmedizin als vorteilhaft erwiesen, den oder die erfindungsgemäßen Wirkstoffe in Gesamtmengen von 0.1 bis 200 mg/kg, vorzugsweise 1 bis 100 mg/kg Körpergewicht je 24 Stunden, gegebenenfalls in Form mehrerer Einzelgaben, zur Erzielung des gewünschten Ergebnisses zu verabreichen. Eine Einzelgabe enthält den oder die Wirkstoffe vorzugsweise in Mengen von 1 bis 80 mg/kg, insbesondere 1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

## A) Beispiele

### Abkürzungen:

| | |
|---|---|
| aq. | wässrig, wässrige Lösung |
| DCI | direkte chemische Ionisation (bei MS) |
| DMA | *N*,*N*-Dimethylacetamid |
| DME | 1,2-Dimethoxyethan |
| DMF | *N*,*N*-Dimethylformamid |
| DMSO | Dimethylsulfoxid |
| d. Th. | der Theorie (bei Ausbeute) |
| EDC | *N'*-(3-Dimethylaminopropyl)-*N*-ethylcarbodiimid x HCl |
| eq. | Äquivalent(e) |
| ESI | Elektrospray-Ionisation (bei MS) |
| h | Stunde(n) |
| HATU | *O*-(7-Azabenzotriazol-1-yl)-*N*,*N*,*N'*,*N'*-tetramethyluronium-hexafluorophosphat |
| HPLC | Hochdruck-, Hochleistungsflüssigchromatographie |
| i. Vak. | im Vakuum |
| konz. | konzentriert |
| LC-MS | Flüssigchromatographie-gekoppelte Massenspektroskopie |
| min | Minute(n) |
| MS | Massenspektroskopie |
| NMR | Kernresonanzspektroskopie |
| PyBOP | Benzotriazol-1-yloxytris(pyrrolidino)phosphonium-hexafluorophosphat |
| $R_t$ | Retentionszeit (bei HPLC) |
| RT | Raumtemperatur |
| TBTU | *O*-(Benzotriazol-1-yl)-*N*,*N*,*N'*,*N'*-tetramethyluronium-tetrafluoroborat |
| TFA | Trifluoressigsäure |
| THF | Tetrahydrofuran |
| TMOF | Trimethylorthoformiat |

### LC-MS/GC-MS-Methoden:

Methode 1:

Gerätetyp MS: Micromass ZQ; Gerätetyp HPLC: HP 1100 Series; UV DAD; Säule: Phenomenex Gemini 3μ 30 mm x 3.00 mm; Eluent A: 1 1 Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 2:

Instrument: Micromass Quattro LCZ mit HPLC Agilent Serie 1100; Säule: Phenomenex Synergi 2μ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A - 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

Methode 3:

Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 4:

Instrument: Micromass Quattro LCZ mit HPLC Agilent Serie 1100; Säule: Phenomenex Onyx Monolithic C18, 100 mm x 3 mm. Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 5:

Instrument: Micromass QuattroPremier mit Waters UPLC Acquity; Säule: Thermo Hypersil GOLD 1.9μ 50 mm x 1mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 1.5 min 10%A → 2.2 min 10%A Ofen: 50°C; Fluss: 0.33 ml/min; UV-Detektion: 210 nm.

Methode 6:

Gerätetyp MS: Waters ZQ; Gerätetyp HPLC: Waters Alliance 2795; Säule: Phenomenex Onyx Monolithic C18, 100 mm x 3 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 7:

Gerätetyp MS: Micromass ZQ; Gerätetyp HPLC: Waters Alliance 2795; Säule: Phenomenex Synergi 2.5 μ MAX-RP 100A Mercury 20 mm x 4mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

Methode 8:

Instrument: Micromass Quattro LCZ mit HPLC Agilent Serie 1100; Säule: Phenomenex Gemini 3μ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

Methode 9:

Instrument: Micromass Quattro LCZ mit HPLC Agilent Serie 1100; Säule: Phenomenex Synergi 2.5 μ MAX-RP 100A Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208-400 nm.

Methode 10:

Gerätetyp MS: Waters (Micromass) Quattro Micro; Gerätetyp HPLC: Agilent 1100 Serie; Säule: Thermo Hypersil GOLD 3μ 20mm x 4mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 3.0 min 10%A → 4.0 min 10%A → 4.01 min 100%A (Flow 2.5ml) → 5.00 min 100%A; Ofen: 50°C; Fluss: 2 ml/min; UV-Detektion: 210 nm.

Methode 11:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (3 min halten).

Methode 12:

Instrument: Waters ACQUITY SQD UPLC System; Säule: Waters Acquity UPLC HSS T3 1.8 μ 50 x 1 mm; Eluent A: 1 l Wasser + 0.25 ml 99%ige Ameisensäure, Eluent B: 1 l Acetonitril + 0.25 ml 99%ige Ameisensäure; Gradient:

0.0 min 90% A → 1.2 min 5% A → 2.0 min 5% A Ofen: 50°C; Fluss: 0.40 ml/min; UV-Detektion: 210 - 400 nm.

**Ausgangsverbindungen und Intermediate:**

**Beispiel 1A**

2-Brom-1-(3-chlor-4-fluorphenyl)ethanon

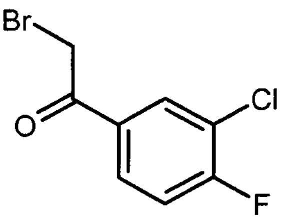

1.00 g (5.79 mmol) 3-Chlor-4-fluoracetophenon wird bei 0°C in 20 ml einer 1:1-Mischung aus 1,4-Dioxan und Diethylether vorgelegt, unter Lichtausschluss tropfenweise mit einer Lösung aus 0.30 ml (5.79 mmol) Brom in 10 ml einer 1:1-Mischung aus 1,4-Dioxan und Diethylether versetzt und 4 Stunden gerührt. Anschließend gibt man Wasser und Dichlormethan hinzu, trennt die Phasen, extrahiert die wässrige Phase mit Dichlormethan, trocknet die vereinigten organischen Phasen über Natriumsulfat, filtriert und engt ein. Man erhält 1.35 g mit 68%iger Reinheit (63% d. Th.) der Titelverbindung, die ohne weitere Aufreinigung umgesetzt werden.
$^1$H-NMR (400 MHz, DMSO-$d_6$): $\delta$ = 8.23 (dd, 1H), 8.04 (ddd, 1H), 7.63 (t, 1H), 4.97 (s, 2H).
GC-MS (Methode 11): $R_t$ = 5.22 min; MS (EIpos): m/z = 252 $[M]^+$.

**Beispiel 2A**

4-(3-Chlor-4-fluorphenyl)-1,3-thiazol-2-carbonsäureethylester

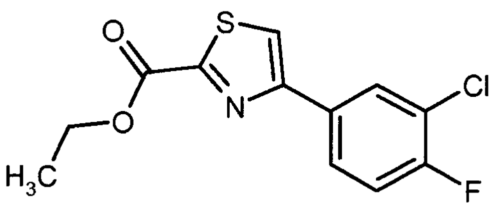

1.35 g der Verbindung aus Beispiel 1A mit 68%iger Reinheit (5.37 mmol) werden in 25 ml Ethanol unter Rückfluss erhitzt, tropfenweise mit einer Lösung aus 0.72 g (5.37 mmol) Ethylamino(thioxo)acetat in 10 ml Ethanol versetzt und 18 Stunden unter Rückfluss erhitzt. Man engt das Reaktionsgemisch ein, reinigt den Rückstand mittels Flash-Chromatographie (Laufmittel: Cyclohexan/Essigsäureethylester 10:1) und erhält 1.94 g der Titelverbindung mit 57%iger Reinheit (100% d. Th.).
$^1$H-NMR (400 MHz, DMSO-$d_6$): $\delta$ = 8.65 (s, 1H), 8.21 (dd, 1H), 8.03 (ddd, 1H), 7.55 (t, 1H), 4.42 (q, 2H), 1.36 (t, 3H).
LC-MS (Methode 5): $R_t$ = 1.15 min; MS (ESIpos): m/z = 258 $[M+H]^+$.

**Beispiel 13A**

4-(3-Chlorphenyl)-1,3-thiazol-2-carbonsäureethylester

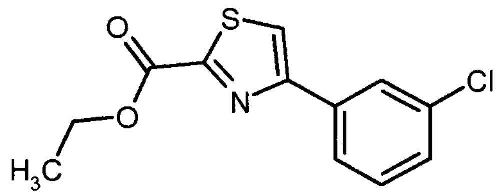

Die Herstellung der Titelverbindung erfolgt ausgehend von 2-Brom-1-(3-chlorphenyl)ethanon analog zur Synthese der Verbindung aus Beispiel 2A. Man erhält 1.51 g (73% d. Th.) der Titelverbindung.
$^1$H-NMR (400 MHz, DMSO-$d_6$): $\delta$ = 8.68 (s, 1H), 8.07 (t, 1H), 7.99 (d, 1H), 7.53 (t, 1H), 7.48 (d, 1H), 4.43 (q, 2H), 1.37 (t, 3H).
LC-MS (Methode 1): $R_t$ = 2.69 min; MS (ESIpos): m/z = 268 $[M+H]^+$.

**Beispiel 4A**

5-Brom-4-(3-chlor-4-fluorphenyl)-1,3-thiazol-2-carbonsäureethylester

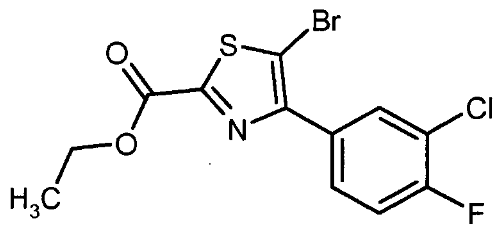

1.94 g der Verbindung aus Beispiel 2A mit 57%iger Reinheit (3.87 mmol) werden in 35 ml Dichlormethan vorgelegt, mit 6.04 g (33.9 mmol) N-Bromsuccinimid und einer Spatelspitze Eisentrichlorid versetzt und 18 Stunden unter Rückfluss erhitzt. Man engt das Reaktionsgemisch ein, reinigt den Rückstand mittels Flash-Chromatographie (Laufmittel: Cyclohexan/Essigsäureethylester 30:1) und erhält 1.30 g (83% d. Th.) der Titelverbindung.
$^1$H-NMR (400 MHz, DMSO-$d_6$): $\delta$ = 8.04 (dd, 1H), 7.90 (ddd, 1H), 7.61 (t, 1H), 4.42 (q, 2H), 1.35 (t, 3H).
LC-MS (Methode 5): $R_t$ = 1.52 min; MS (ESIpos): m/z = 364 $[M+H]^+$.

**Beispiel SA**

5-Brom-4-(3-chlorphenyl)-1,3-thiazol-2-carbonsäureethylester

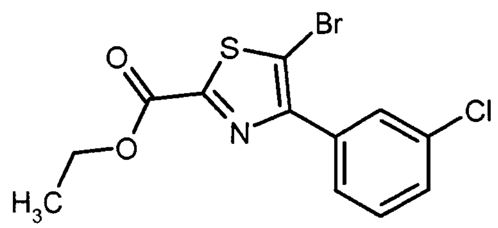

Die Herstellung der Titelverbindung erfolgt ausgehend von der Verbindung aus Beispiel 3A analog zur Synthese der Verbindung aus Beispiel 4A. Man erhält 1.95 g der Titelverbindung mit 65%iger Reinheit (98% d. Th.).
LC-MS (Methode 1): $R_t$ = 3.01 min; MS (ESIpos): m/z = 346 $[M+H]^+$.

**Beispiel 6A**

5-Brom-4-(3-chlor-4-fluorphenyl)-1,3-thiazol-2-carbonsäure

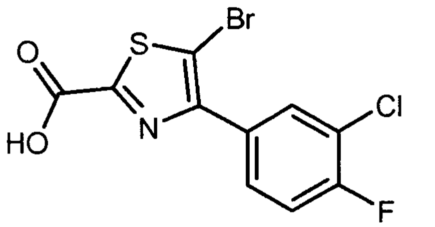

400 mg (1.10 mmol) der Verbindung aus Beispiel 4A werden in 19 ml Tetrahydrofuran vorgelegt und bei Raumtemperatur mit 263 mg (11.0 mmol) Lithiumhydroxid und 19 ml Wasser versetzt. Man rührt bei Raumtemperatur über Nacht, gibt anschließend 1N wässrige Hydrogenchlorid-Lösung bis zu einem sauren pH-Wert hinzu, extrahiert mit Essigsäureethylester, wäscht die organische Phase mit Wasser, trocknet über Natriumsulfat, filtriert und engt ein. Man erhält 190 mg der Titelverbindung mit 86%iger Reinheit (44% d. Th.).
LC-MS (Methode 1): $R_t$ = 2.77 min; MS (ESIpos): m/z = 336 $[M+H]^+$.

**Beispiel 7A**

1-{[5-Brom-4-(3-chlor-4-fluorphenyl)-1,3-thiazol-2-yl]carbonyl}imidazolidin-4-on

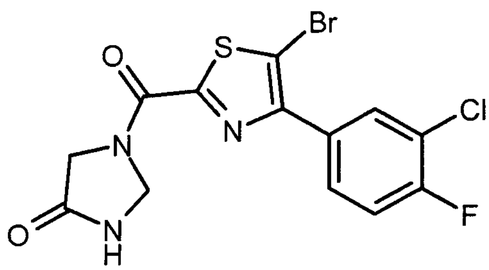

190 mg der Verbindung aus Beispiel 6A mit 86%iger Reinheit (0.49 mmol), 107 mg (0.54 mmol) der Verbindung aus Beispiel 20A und 380 mg (0.73 mmol) PyBOP werden in 2 ml Tetrahydrofuran vorgelegt und bei Raumtemperatur mit 0.27 ml (1.56 mmol) N,N-Diisopropylethylamin versetzt. Man rührt bei Raumtemperatur über Nacht und reinigt anschließend die Reaktionsmischung über präparative HPLC (RP18-Säule; Laufmittel: Acetonitril/Wasser-Gradient unter Zusatz von 0.1% Ameisensäure). Man erhält 109 mg (55% d. Th.) der Titelverbindung.
$^1$H-NMR (400 MHz, DMSO-$d_6$): $\delta$ = 8.80 (s, 0.6H), 8.71 (s, 0.4H), 8.16-8.09 (m, 1H), 8.01-7.93 (m, 1H), 7.66-7.59 (m, 1H), 5.45 (s, 0.8H), 4.91 (s, 1.2H), 4.55 (s, 1.2H), 4.00 (s, 0.8H).
LC-MS (Methode 7): $R_t$ = 1.86 min; MS (ESIpos): m/z = 404 $[M+H]^+$.

**Beispiel 8A**

4-(3-Chlor-4-fluorphenyl)-5-(3-chlor-5-fluorphenyl)-1,3-thiazol-2-carbonsäure

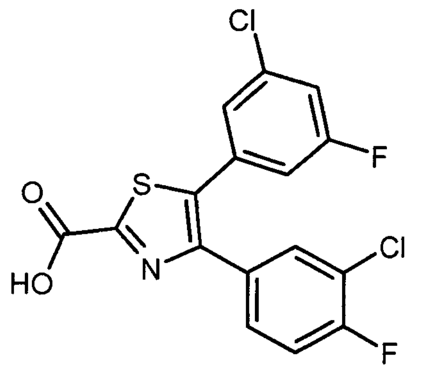

100 mg (0.27 mmol) der Verbindung aus Beispiel 4A werden unter Argon in 4 ml 1,2-Dimethoxyethan vorgelegt und mit 71.7 mg (0.41 mmol) 3-Chlor-5-fluorphenylboronsäure, 1.5 ml (1.37 mmol) wässriger NatriumhydrogencarbonatLösung (10%ig) und 9.5 mg (8μmol) Tetrakis(triphenylphosphin)palladium(0) versetzt. Es wird bei 80°C über Nacht gerührt. Man engt ein, reinigt den Rückstand über präparative HPLC (RP18-Säule; Laufmittel: Acetonitril/Wasser-Gradient) und erhält 93.0 mg (88% d. Th.) der Titelverbindung.
$^1$H-NMR (400 MHz, DMSO-$d_6$): $\delta$ = 7.67 (dd, 1H), 7.48 (dt, 1H), 7.38 (t, 1H), 7.31 (ddd, 1H), 7.28 (t, 1H), 7.23 (ddd, 1H).
LC-MS (Methode 1): $R_t$ = 3.57 min; MS (ESIpos): m/z = 386 $[M+H]^+$.

**Beispiel 9A**

4-(3-Chlor-4-fluorphenyl)-5-(3-chlorphenyl)-1,3-thiazol-2-carbonsäure

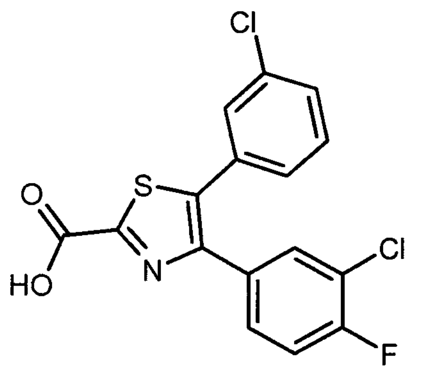

Die Herstellung der Titelverbindung erfolgt ausgehend von der Verbindung aus Beispiel 4A analog zur Synthese der Verbindung aus Beispiel 8A. Man erhält 80.0 mg (79% d. Th.) der Titelverbindung.
$^1$H-NMR (400 MHz, DMSO-$d_6$): $\delta$ = 7.65 (dd, 1H), 7.48-7.28 (m, 6H).
LC-MS (Methode 7): $R_t$ = 2.14 min; MS (ESIpos): m/z = 368 $[M+H]^+$.

**Beispiel 10A**

5-(3-Chlor-5-fluorphenyl)-4-(3-chlorphenyl)-1,3-thiazol-2-carbonsäure

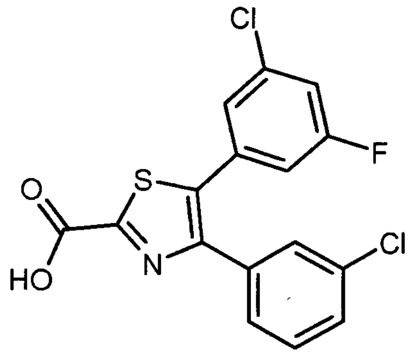

Die Herstellung der Titelverbindung erfolgt ausgehend von der Verbindung aus Beispiel 5A analog zur Synthese der Verbindung aus Beispiel 8A. Man erhält 83.0 mg (78% d. Th.) der Titelverbindung.
$^1$H-NMR (400 MHz, DMSO-$d_6$): $\delta$ = 7.55-7.53 (m, 1H), 7.48 (dt, 1H), 7.39-7.33 (m, 2H), 7.30-7.24 (m, 2H), 7.24-7.19 (m, 1H).
LC-MS (Methode 1): $R_t$ = 3.33 min; MS (ESIpos): m/z = 368 $[M+H]^+$.

**Beispiel 11A**

5-(3-Chlor-4-fluorphenyl)-4-(3-chlorphenyl)-1,3-thiazol-2-carbonsäure

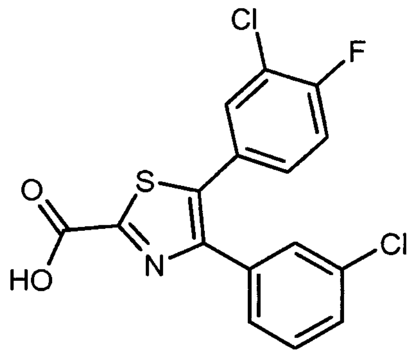

Die Herstellung der Titelverbindung erfolgt ausgehend von der Verbindung aus Beispiel 5A analog zur Synthese der Verbindung aus Beispiel 8A. Man erhält 59.0 mg (56% d. Th.) der Titelverbindung.
$^1$H-NMR (400 MHz, DMSO-$d_6$): $\delta$ = 7.58 (dd, 1H), 7.55-7.52 (m, 1H), 7.45 (t, 1H), 7.36-7.30 (m, 3H), 7.26 (dt, 1H).
LC-MS (Methode 1): $R_t$ = 3.26 min; MS (ESIpos): m/z = 368 $[M+H]^+$.

**Beispiel 12A**

4-(3-Chlorphenyl)-5-(3-cyan-4-fluorphenyl)-1,3-thiazol-2-carbonsäure

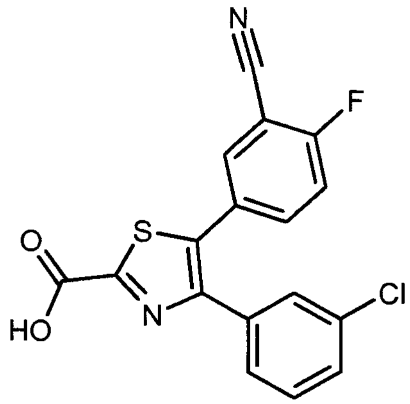

Die Herstellung der Titelverbindung erfolgt ausgehend von der Verbindung aus Beispiel 5A analog zur Synthese der Verbindung aus Beispiel 8A. Man erhält 42.4 mg (57% d. Th.) der Titelverbindung.
LC-MS (Methode 7): $R_t$ = 2.23 min; MS (ESIpos): m/z = 359 $[M+H]^+$.

**Beispiel 13A**

4-(3-Chlorphenyl)-5-(3-cyanphenyl)-1,3-thiazol-2-carbonsäure

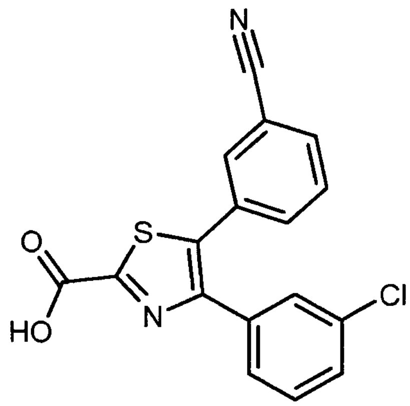

Die Herstellung der Titelverbindung erfolgt ausgehend von der Verbindung aus Beispiel 5A analog zur Synthese der Verbindung aus Beispiel 8A. Man erhält 556 mg der Titelverbindung mit 41%iger Reinheit (46% d. Th.).
LC-MS (Methode 10): $R_t$ = 2.13 min; MS (ESIpos): m/z = 341 $[M+H]^+$.

**Beispiel 14A**

4-(3-Chlorphenyl)-5-[3-(trifluormethyl)phenyl]-1,3-thiazol-2-carbonsäure

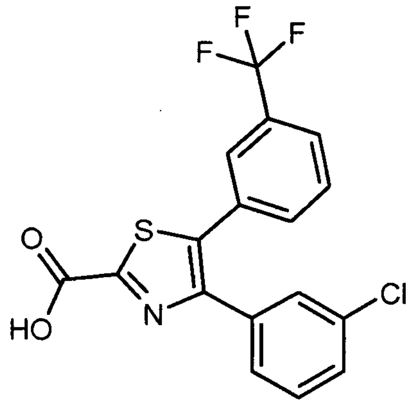

Die Herstellung der Titelverbindung erfolgt ausgehend von der Verbindung aus Beispiel 5A analog zur Synthese der Verbindung aus Beispiel 8A. Man erhält 246 mg der Titelverbindung mit 64%iger Reinheit (55% d. Th.).
LC-MS (Methode 5): $R_t$ = 1.27 min; MS (ESIpos): m/z = 384 $[M+H]^+$.

**Beispiel 15A**

4-(3-Chlor-4-fluorphenyl)-5-(3-cyan-4-fluorphenyl)-1,3-thiazol-2-carbonsäure

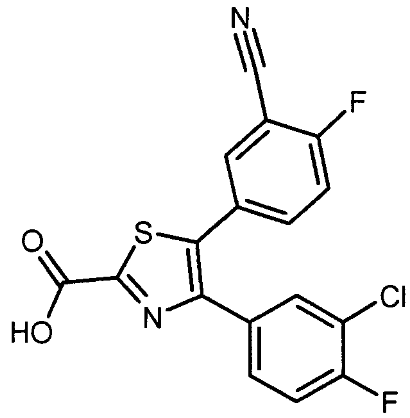

Die Herstellung der Titelverbindung erfolgt ausgehend von der Verbindung aus Beispiel 4A analog zur Synthese der Verbindung aus Beispiel 8A. Man erhält 142 mg der Titelverbindung mit 85%iger Reinheit (58% d. Th.).
LC-MS (Methode 7): $R_t$ = 2.27 min; MS (ESIpos): m/z = 377 $[M+H]^+$.

**Beispiel 16A**

4-(3-Chlor-4-fluorphenyl)-5-(3-cyanphenyl)-1,3-thiazol-2-carbonsäure

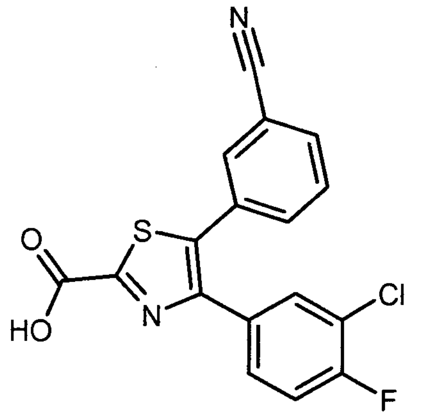

Die Herstellung der Titelverbindung erfolgt ausgehend von der Verbindung aus Beispiel 4A analog zur Synthese der Verbindung aus Beispiel 8A. Man erhält 161 mg (76% d. Th.) der Titelverbindung.
LC-MS (Methode 7): $R_t$ = 2.18 min; MS (ESIpos): m/z = 359 $[M+H]^+$.
$^1$H-NMR (400 MHz, DMSO-$d_6$): $\delta$ = 14.4 (s, 1H), 8.00 (t, 1H), 7.95 (dt, 1H), 7.73 (dt, 1H), 7.68-7.62 (m, 2H), 7.43 (t, 1H), 7.34 (ddd, 1H).

**Beispiel 17A**

$N^2$-Benzylglycinamid

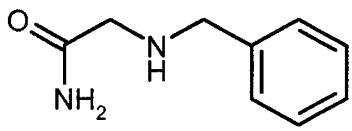

44.2 g (0.40 mol) Glycinamid-hydrochlorid werden in 2.2 1 Dichlormethan bei Raumtemperatur unter Argon vorgelegt, mit 112 ml (0.80 mol) Triethylamin versetzt und über Nacht bei Raumtemperatur gerührt. Dann werden 42.5 g (0.40 mol) Benzaldehyd zugegeben und über Nacht am Wasserabscheider unter Rückfluss erhitzt. Man engt ein, löst den Rückstand in 400 ml Tetrahydrofuran/Methanol (1:1), versetzt bei 0°C portionsweise mit 16.7 g (0.44 mol) Natriumborhydrid und rührt zwei Tage bei Raumtemperatur. Die Suspension wird abgesaugt, das Filtrat eingeengt und im Hochvakuum getrocknet. Der Rückstand wird in Essigsäureethylester gerührt, der Niederschlag abfiltriert, das Filtrat eingeengt und der Rückstand in Toluol über Nacht gerührt. Nach Filtration des Feststoffs und anschließendem Trocknen im Hochvakuum erhält man 56.5 g (84% d. Th.) der Titelverbindung.
$^1$H-NMR (400 MHz, DMSO-$d_6$): $\delta$ = 7.36-7.28 (m, 4H), 7.27-7.19 (m, 1H), 3.68-3.64 (m, 2H), 3.03-3.00 (m, 2H).
LC-MS (Methode 10): $R_t$ = 0.40 min; MS (ESIpos): m/z = 165 $[M+H]^+$.

**Beispiel 18A**

1-Benzyl-3-(hydroxymethyl)imidazolidin-4-on

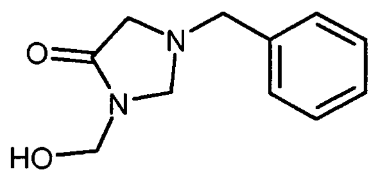

56.5 g (0.34 mol) der Verbindung aus Beispiel 17A werden mit 172 ml (6.20 mol) 37%iger Formaldehyd-Lösung versetzt

und 30 Minuten unter Rückfluss erhitzt. Es wird mit Dichlormethan extrahiert, die vereinigten organischen Phasen über Natriumsulfat getrocknet, filtriert und eingeengt. Man erhält 74.5 g (100% d. Th.) der Titelverbindung.
LC-MS (Methode 5): $R_t$ = 0.51 min; MS (ESIpos): m/z = 207 $[M+H]^+$.

**Bespiel 19A**

1-Benzylimidazolidin-4-on-trifluoracetat

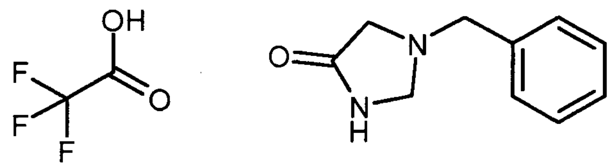

74.5 g (0.36 mol) der Verbindung aus Beispiel 18A werden für 6 h bei 150°C im Hochvakuum unter Abdestillieren flüchtiger Reaktionsprodukte erhitzt. Die Aufreinigung des Rückstands erfolgt durch HPLC (Säule: Sunfire C18 5$\mu$, 250 x 20 mm;
Eluent: 0.2% Trifluoressigsäure/Wasser-Acetonitril-Gradient). Man erhält 28.4 g (27% d. Th.) der Titelverbindung.
$^1$H-NMR (400 MHz, DMSO-$d_6$): $\delta$ = 8.75 (s, 1H), 7.48-7.39 (m, 5H), 4.41 (s, 2H), 4.22 (s, 2H), 3.54 (s, 2H).
LC-MS (Methode 10): $R_t$ = 0.94 min; MS (ESIpos): m/z = 177 $[M-CF_3COOH+H]^+$.

**Beispiel 20A**

Imidazolidin-4-on-trifluoracetat

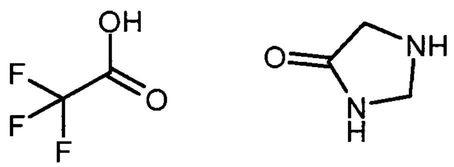

28.4 g (97.9 mmol) der Verbindung aus Beispiel 19A werden in 750 ml Ethanol gelöst und unter Argon mit 4.5 g (42.3 mmol) Palladium auf Aktivkohle (5%ig) versetzt. Es wird 24 h bei Raumtemperatur unter Wasserstoffatmosphäre gerührt. Die Suspension wird über Celite filtriert, eingeengt und am Hochvakuum getrocknet. Man erhält 19.2 g (98% d. Th.) der Titelverbindung.
$^1$H-NMR (400 MHz, DMSO-$d_6$): $\delta$ = 10.1 (s, 2H), 8.89 (s, 1H), 4.55 (s, 2H), 3.63 (s, 2H).
GC-MS (Methode 11): $R_t$ = 3.92 min MS (EIpos): m/z = 86 $[M-CF_3COOH]^+$.

**Beispiel 21A**

3-Fluor-5-(4,4,5,5-tetramethyl-1,3,2-dioxaborolan-2-yl)benzolcarbonitril

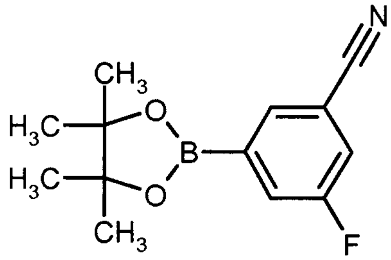

3.60 g (18.0 mmol) 3-Brom-5-fluorbenzolcarbonitril, 5.03 g (19.8 mmol) 4,4,4',4',5,5,5',5'-Octamethyl-2,2'-bi-1,3,2-dioxaborolan und 5.30 g (54.0 mmol) Kaliumacetat werden unter Argon in 72 ml entgastem 1,4-Dioxan/DMSO (10/1) vorgelegt und mit 441 mg (0.54 mmol) 1,1'-Bis(diphenylphosphin)ferrocendichlorpalladium(II)-Dichlormethan-Komplex versetzt. Es wird über Nacht bei 90°C gerührt. Anschließend gibt man Wasser hinzu, trennt die Phasen, extrahiert die wässrige Phase mit Essigsäureethylester und engt die vereinigten organischen Phasen ein. Das Rohprodukt wird mittels Flash-Chromatographie (Laufmittel: Cyclohexan/Essigsäureethylester 10:1) gereinigt. Man erhält 4.48 g (92% d. Th.) der Titelverbindung.
$^1$H-NMR (400 MHz, DMSO-$d_6$): $\delta$ = 8.01 (ddd, 1H), 7.82 (s, 1H), 7.70 (ddd, 1H), 1.32 (s, 12H).
GC-MS (Methode 11): $R_t$ = 4.94 min; MS (EIpos): m/z = 247 $[M]^+$.

**Beispiel 22A**

(3-Cyan-5-fluorphenyl)boronsäure

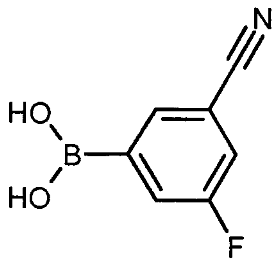

1.00 g (4.05 mmol) der Verbindung aus Beispiel 21A werden in 40 ml Aceton vorgelegt, mit 2.60 g (12.1 mmol) Natriumperiodat, 0.70 g (9.11 mmol) Ammoniumacetat und 40 ml Wasser versetzt und über Nacht bei Raumtemperatur gerührt. Anschließend engt man ein, versetzt den Rückstand mit 2N wässriger NatriumhydroxidLösung und saugt den verbliebenen Niederschlag ab. Die Lösung wird mit konzentrierter wässriger Hydrogenchlorid-Lösung auf einen pH-Wert von 3 gestellt und auf 0°C gekühlt. Der entstehende Niederschlag wird abgesaugt, mit Wasser gewaschen und im Hochvakuum getrocknet. Man erhält 384 mg (58% d. Th.) der Titelverbindung.
$^1$H-NMR (400 MHz, DMSO-$d_6$): $\delta$ = 8.57 (s, 2H), 7.98 (s, 1H), 7.88 (ddd, 1H), 7.84 (dd, 1H).
LC-MS (Methode 7): $R_t$ = 1.07 min; MS (ESIneg): m/z = 164 $[M-H]^-$.

**Beispiel 23A**

3-Acetyl-5-fluorbenzolcarbonitril

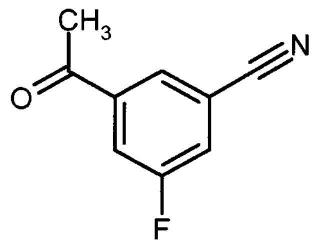

9.00 g (45.0 mmol) 3-Brom-5-fluorbenzolcarbonitril in Toluol (300 ml) werden unter einer Argonatmosphäre mit 824 mg (0.900 mmol) Tris(dibenzylidenaceton)dipalladium und 1.23 g (1.98 mmol) rac-1,1'-Binaphthalen-2,2'-diylbis(diphenylphosphan) versetzt. Nach Zugabe von 19.5 g (54.0 mmol) (1-Ethoxyvinyl)-tributylstannan wird unter Rückfluss über Nacht gerührt. Anschließend wird die Reaktionsmischung eingeengt und der Rückstand in 300 ml THF aufgenommen. Nach Zugabe von 100 ml wässriger 2 N Hydrogenchlorid-Lösung wird 2 h bei Raumtemperatur gerührt. Die Reaktionsmischung wird anschließend mit gesättigter wässriger Natriumhydrogencarbonat-Lösung neutralisiert und mit Ethylacetat extrahiert. Die vereinigten organischen Phasen werden über Magnesiumsulfat getrocknet, filtriert und i. Vak. eingeengt. Das Rohprodukt wird mittels Flash-Chromatographie (Laufmittel: Cyclohexan/Essigsäureethylester 9:1) gereinigt. Man erhält 7.11 g (97% d. Th.) der Titelverbindung.

$^1$H-NMR (400 MHz, DMSO-$d_6$): $\delta$ = 8.28 (t, 1H), 8.18-8.14 (m, 1H), 8.08-8.04 (m, 1H), 2.65 (s, 3H).

GC-MS (Methode 11): $R_t$ = 3.97 min; MS (EIpos): m/z = 163 $[M]^+$.

**<u>Beispiel 24A</u>**

4-(3-Cyan-5-fluorphenyl)-1,3-thiazol-2-carbonsäureethylester

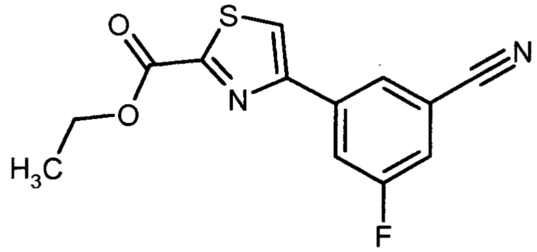

1.40 g (8.58 mmol) der Verbindung aus Beispiel 23A in 15 ml konz. Essigsäure werden bei Raumtemperatur mit 5 Tropfen einer konz. wässrigen Hydrogenchlorid-Lösung versetzt. Anschließend werden 0.44 ml (8.58 mmol) Brom in 4 ml konz. Essigsäure innerhalb 0.5 h zugetropft. Anschließend werden weitere 0.20 ml (3.90 mmol) Brom in 2 ml konz. Essigsäure zugetropft, und die Reaktionsmischung wird 15 min gerührt und dann auf Eis gegossen. Nach Extraktion der wässrigen Phase mit Ethylacetat werden die vereinigten organischen Phasen über $MgSO_4$ getrocknet, filtriert und i. Vak. eingeengt. 2.24 g des erhaltenen Feststoffes werden in 90 ml EtOH vorgelegt, unter Rückfluss erhitzt und tropfenweise mit einer Lösung aus 1.23 g (9.25 mmol) Ethylamino(thioxo)acetat in 10 ml Ethanol versetzt und 3 Stunden unter Rückfluss gerührt. Die Reaktionslösung wird abgekühlt und der entstandene Niederschlag abfiltriert. Die Mutterlage wird i. Vak. eingeengt, der Rückstand in wenig Ethanol aufgenommen und anschließend der entstandene Feststoff abfiltriert. Man erhält nach Vereinigung der Feststoffe 1.52 g (59% d. Th.) der Titelverbindung.

$^1$H-NMR (400 MHz, DMSO-$d_6$): $\delta$ = 8.82 (s, 1H), 8.33 (s, 1H), 8.18 (d, 1H), 7.91 (d, 1H), 4.43 (q, 2H), 1.37 (t, 3H).

LC-MS (Methode 1): $R_t$ = 2.35 min; MS (ESIpos): m/z = 277 $[M+H]^+$.

**<u>Beispiel 25A</u>**

5-Brom-4-(3-cyan-5-fluorphenyl)-1,3-thiazol-2-carbonsäureethylester

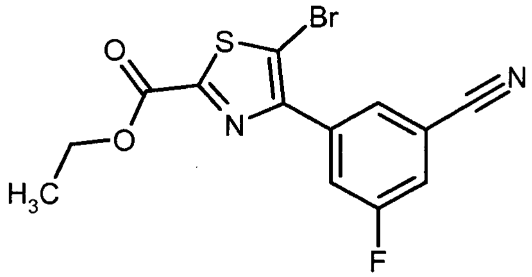

2.21 g (7.98 mmol) der Verbindung aus Beispiel 24A in 75 ml konz. Essigsäure werden bei Raumtemperatur mit 12.8 g (79.8 mmol) Brom und 7.83 g (79.8 mmol) Kaliumacetat versetzt. Es wird für 12 h bei 100°C gerührt, wobei nach 6 und 9 h nochmals gleiche Mengen an Brom (jeweils 12.8 g, 79.8 mmol) und Kaliumacetat (jeweils 7.83 g, 79.8 mmol) zugefügt werden. Anschließend wird die Reaktionslösung mit 1 M wässriger Natriumsulfit-Lösung versetzt und mit Dichlormethan extrahiert. Die vereinigten organischen Phasen werden über $MgSO_4$ getrocknet, filtriert und i. Vak. eingeengt. Das Rohprodukt wird mittels Flash-Chromatographie (Laufmittel: Cyclohexan/Essigsäureethylester 20:1→10:1) gereinigt. Man erhält 2.31 g (78% d. Th.) der Titelverbindung.
$^1$H-NMR (400 MHz, DMSO-$d_6$): $\delta$ = 8.15 (s, 1H), 8.05 (d, 1H), 8.03 (d, 1H), 4.42 (q, 2H), 1.35 (t, 3H).
LC-MS (Methode 1): $R_t$ = 2.58 min; MS (ESIpos): m/z = 357 $[M+H]^+$.

**Beispiel 26A**

5-(3-Chlor-5-fluorphenyl)-4-(3-cyan-5-fluorphenyl)-1,3-thiazol-2-carbonsäure

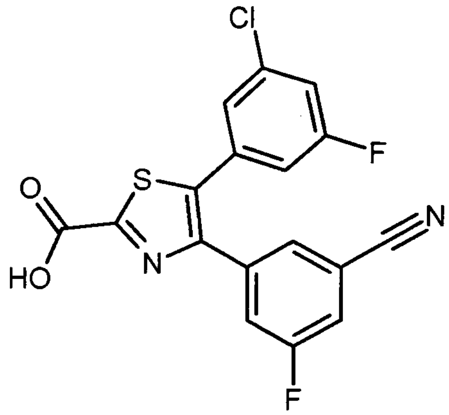

400 mg (1.13 mmol) der Verbindung aus Beispiel 25A und 65.1 mg (0.056 mmol) Tetrakis(triphenylphosphin)palladium in 29 ml DME werden bei Raumtemperatur mit 295 mg (1.69 mmol) der Boronsäure aus Beispiel 22A versetzt. Anschließend werden 289 mg (3.44 mmol) Natriumhydrogencarbonat in 13 ml Wasser zugeben und die Mischung wird für 1 h unter Rückfluss gerührt. Die Reaktionslösung wird anschließend i. Vak. eingeengt, der Rückstand in Ethylacetat aufgenommen und mit gesättigter wässriger Natriumchlorid-Lösung gewaschen. Die organische Phase wird über Magesiumsulfat getrocknet, filtriert und eingeengt. Der Rückstand wird über präparative HPLC (RP18-Säule; Laufmittel: Acetonitril/Wasser-Gradient) gereinigt. Man erhält 228 mg (45% d. Th.) der Titelverbindung in 83%iger Reinheit.
LC-MS (Methode 5): $R_t$ = 1.17 min; MS (ESIpos): m/z = 377 $[M+H]^+$.

**Beispiel 27A**

3-Acetyl-6-fluorbenzolcarbonitril

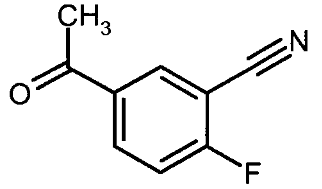

4.90 g (24.5 mmol) 3-Brom-6-fluorbenzolcarbonitril in 180 ml Toluol werden unter einer Argonatmosphäre mit 449 mg (0.490 mmol) Tris(dibenzylidenaceton)dipalladium und 671 mg (1.078 mmol) rac-1,1'-Binaphthalen-2,2'-diylbis(diphenylphosphan) versetzt. Nach Zugabe von 10.6 g (29.4 mmol) (1-Ethoxyvinyl)-tributylstannan wird unter Rückfluss über Nacht gerührt. Anschließend wird die Reaktionsmischung eingeengt und der Rückstand in 200 ml THF aufgenommen. Nach Zugabe von wässriger 2 N Hydrogenchlorid-Lösung (50 ml) wird 4 h bei Raumtemperatur gerührt. Die Reaktionsmischung wird anschließend mit gesättigter wässriger Natriumhydrogencarbonat-Lösung neutralisiert und mit Ethylacetat extrahiert. Die vereinigten organischen Phasen werden über Magnesiumsulfat getrocknet, filtriert und i. Vak. eingeengt. Das Rohprodukt wird mittels Flash-Chromatographie (Laufmittel: Cyclohexan/Essigsäureethylester 10:1→3:1) gereinigt. Man erhält 3.50 g (88% d. Th.) der Titelverbindung.
$^1$H-NMR (400 MHz, DMSO-$d_6$): δ = 8.55 (dd, 1H), 8.31 (ddd, 1H), 7.68 (t, 1H), 2.63 (s, 3H).
GC-MS (Methode 11): $R_t$ = 4.34 min; MS (EIpos): m/z = 163 $[M]^+$.

**Beispiel 28A**

4-(3-Cyan-4-fluorphenyl)-1,3-thiazol-2-carbonsäureethylester

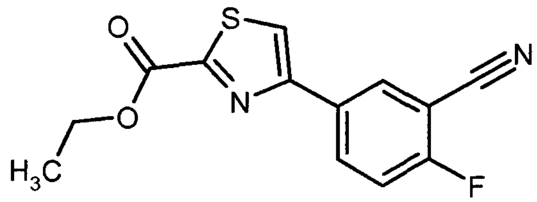

3.44 g (21.1 mmol) der Verbindung aus Beispiel 27A in 50 ml konz. Essigsäure werden bei Raumtemperatur mit 10 Tropfen einer konz. wässrigen Hydrogenchlorid-Lösung versetzt. Nachfolgend werden 1.08 ml (21.1 mmol) Brom in 20 ml konz. Essigsäure innerhalb 1 h zugetropft. Anschließend werden weitere 0.20 ml (3.90 mmol) Brom in 2 ml konz. Essigsäure zugetropft, und die Reaktionsmischung 30 min gerührt und anschließend auf Eis gegossen. Nach Extraktion der wässrigen Phase mit Dichlormethan werden die vereinigten organischen Phasen über $MgSO_4$ getrocknet, filtriert und i. Vak. eingeengt. 5.40 g des erhaltenen Feststoffes werden in 120 ml EtOH vorgelegt, unter Rückfluss erhitzt und tropfenweise mit einer Lösung aus 1.93 g (14.5 mmol) Ethylamino(thioxo)acetat in 20 ml Ethanol versetzt und 5 h unter Rückfluss gerührt. Die Reaktionslösung wird abgekühlt und der entstandene Niederschlag abfiltriert. Die Mutterlage wird i. Vak. eingeengt und der Rückstand in wenig Ethanol aufgenommen und anschließend der entstandene Feststoff abfiltriert. Man erhält nach Vereinigung der Feststoffe 3.80 g (95% d. Th.) der Titelverbindung.
$^1$H-NMR (400 MHz, DMSO-$d_6$): δ = 8.69 (s, 1H), 8.52 (dd, 1H), 8.40 (ddd, 1H), 7.66 (t, 1H), 4.43 (q, 2H), 1.37 (t, 3H).
LC-MS (Methode 5): $R_t$ = 1.19 min; MS (ESIpos): m/z = 277 $[M+H]^+$.

**Beispiel 29A**

5-Brom-4-(3-Cyan-4-fluorphenyl)-1,3-thiazol-2-carbonsäureethylester

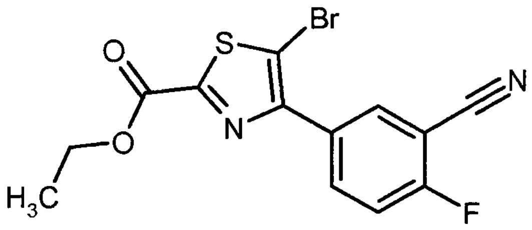

1.40 g (5.07 mmol) der Verbindung aus Beispiel 28A in 80 ml konz. Essigsäure werden bei Raumtemperatur mit 8.10 g (50.6 mmol) Brom und 4.97 g (50.6 mmol) Kaliumacetat versetzt. Es wird für 48 h bei 100°C gerührt, wobei nach 24 und 36 h nochmals Brom (jeweils 4.05 g, 25.3 mmol) und Kaliumacetat (jeweils 2.49 g, 25.3 mmol) zugefügt wird. Anschließend wird die Reaktionslösung mit 1 M wässriger Natriumthiosulfat-Lösung versetzt und mit Dichlormethan extrahiert. Die vereinigten organischen Phasen werden über $MgSO_4$ getrocknet, filtriert und i. Vak. eingeengt. Das Rohprodukt wird mittels Flash-Chromatographie (Laufmittel: Cyclohexan/Essigsäureethylester 20:1→10:1) gereinigt. Man erhält 1.55 g (84% d. Th.) der Titelverbindung.
$^1$H-NMR (400 MHz, DMSO-$d_6$): $\delta$ = 8.37 (dd, 1H), 8.30-8.20 (m, 1H), 7.73 (t, 1H), 4.42 (q, 2H), 1.35 (t, 3H).
LC-MS (Methode 1): $R_t$ = 2.55 min; MS (ESIpos): m/z = 357 $[M+H]^+$.

**Beispiel 30A**

5-(3-Chlor-5-fluorphenyl)-4-(3-cyan-4-fluorphenyl)-1,3-thiazol-2-carbonsäure

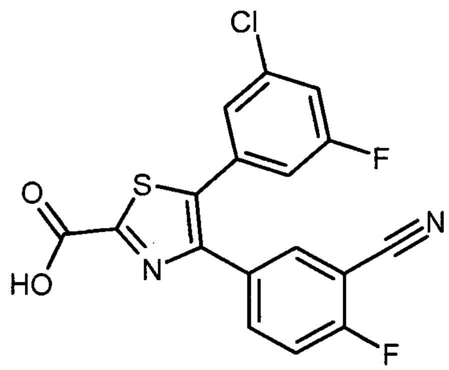

350 mg (0.985 mmol) der Verbindung aus Beispiel 29A und 56.9 mg (0.049 mmol) Tetrakis(triphenylphosphin)palladium in 26 ml DME werden bei Raumtemperatur mit 256 mg (1.48 mmol) (3-Chlor-5-fluorphenyl)boronsäure versetzt. Anschließend werden 252 mg (3.01 mmol) Natriumhydrogencarbonat in 11 ml Wasser zugeben und die Mischung wird für 1 h unter Rückfluss gerührt. Die Reaktionslösung wird anschließend i. Vak. eingeengt, der Rückstand in Ethylacetat aufgenommen und mit gesättigter wässriger Natriumchlorid-Lösung gewaschen. Die organische Phase wird über Magesiumsulfat getrocknet, filtriert und eingeengt. Der Rückstand wird über präparative HPLC (RP18-Säule; Laufmittel: Acetonitril/Wasser-Gradient) gereinigt. Man erhält 50.0 mg (13% d. Th.) und 41 mg (9% d. Th., 84%ige Reinheit) der Titelverbindung.
LC-MS (Methode 7): $R_t$ = 2.15 min; MS (ESIpos): m/z = 377 $[M+H]^+$.

**Beispiel 31A**

5-(3-Chlorphenyl)-4-(3-cyan-4-fluorphenyl)-1,3-thiazol-2-carbonsäure

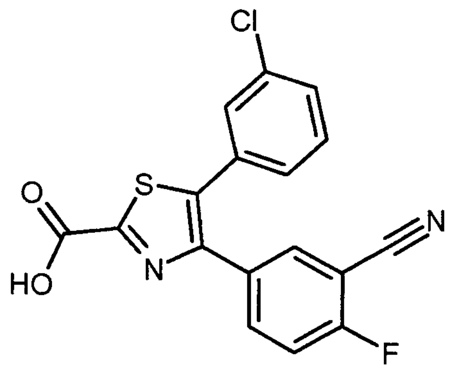

350 mg (0.985 mmol) der Verbindung aus Beispiel 29A und 56.9 mg (0.049 mmol) Tetrakis(triphenylphosphin)palladium in 26 ml DME werden bei Raumtemperatur mit 231 mg (1.48 mmol) (3-Chlorphenyl)boronsäure versetzt. Anschließend werden 252 mg (3.01 mmol) Natriumhydrogencarbonat in 11 ml Wasser zugeben und die Mischung wird für 1 h unter Rückfluss gerührt. Die Reaktionslösung wird anschließend i. Vak. eingeengt, der Rückstand in Ethylacetat aufgenommen und mit gesättigter wässriger Natriumchlorid-Lösung gewaschen. Die organische Phase wird über Magesiumsulfat getrocknet, filtriert und eingeengt. Der Rückstand wird über präparative HPLC (RP18-Säule; Laufmittel: Acetonitril/Wasser-Gradient) gereinigt. Man erhält 353 mg (100% d. Th.) der Titelverbindung.
LC-MS (Methode 5): $R_t$ = 1.17 min; MS (ESIpos): m/z = 359 $[M+H]^+$.

__Beispiel 32A__

4-(3-Cyan-4-fluorphenyl)-5-(3-cyan-5-fluorphenyl)-1,3-thiazol-2-carbonsäure

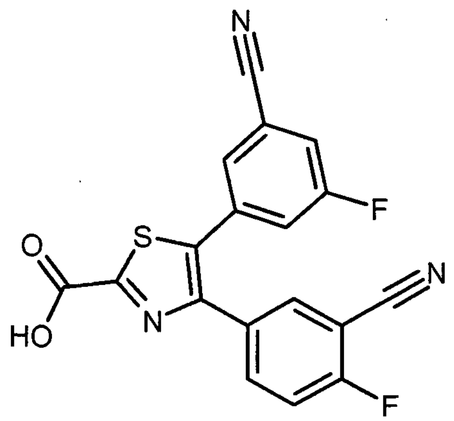

350 mg (0.985 mmol) der Verbindung aus Beispiel 29A und 56.9 mg (0.049 mmol) Tetrakis(triphenylphosphin)palladium in 26 ml DME werden bei Raumtemperatur mit 244 mg (1.48 mmol) der Boronsäure aus Beispiel 22A versetzt. Anschließend werden 252 mg (3.01 mmol) Natriumhydrogencarbonat in 11 ml Wasser zugeben und die Mischung wird für 1 h unter Rückfluss gerührt. Die Reaktionslösung wird anschließend i. Vak. eingeengt, der Rückstand in Ethylacetat aufgenommen und mit gesättigter wässriger Natriumchlorid-Lösung gewaschen. Die organische Phase wird über Magesiumsulfat getrocknet, filtriert und eingeengt. Der Rückstand wird über präparative HPLC (RP18-Säule; Laufmittel: Acetonitril/Wasser-Gradient) gereinigt. Man erhält 97.6 mg (16% d. Th.) der Titelverbindung in 61%iger Reinheit.
LC-MS (Methode 5): $R_t$ = 1.01 min; MS (ESIpos): m/z = 369 $[M+H]^+$.

__Beispiel 33A__

4-(3-Cyan-4-fluorphenyl)-5-(3-cyanphenyl)-1,3-thiazol-2-carbonsäure

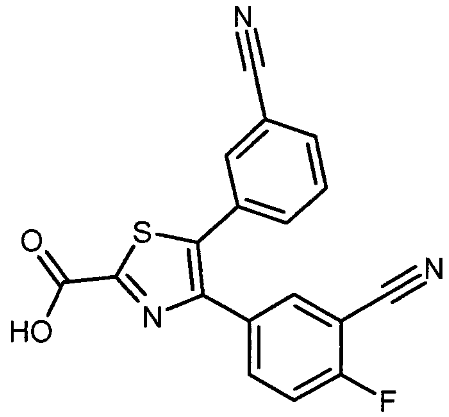

350 mg (0.985 mmol) der Verbindung aus Beispiel 29A und 56.9 mg (0.049 mmol) Tetrakis(triphenylphosphin)palladium in 26 ml DME werden bei Raumtemperatur mit 217 mg (1.48 mmol) (3-Cyanphenyl)boronsäure versetzt. Anschließend werden 252 mg (3.01 mmol) Natriumhydrogencarbonat in 11 ml Wasser zugeben und die Mischung wird für 2 h unter Rückfluss gerührt. Die Reaktionslösung wird anschließend i. Vak. eingeengt, der Rückstand in Ethylacetat aufgenommen und mit gesättigter wässriger Natriumchlorid-Lösung gewaschen. Die organische Phase wird über Magesiumsulfat getrocknet, filtriert und eingeengt. Der Rückstand wird über präparative HPLC (RP18-Säule; Laufmittel: Acetonitril/Wasser-Gradient) gereinigt. Man erhält 47.3 mg (14% d. Th.) der Titelverbindung.
LC-MS (Methode 5): $R_t$ = 0.97 min; MS (ESIpos): m/z = 350 $[M+H]^+$.

**Beispiel 34A**

4-(3-Cyanphenyl)-1,3-thiazol-2-carbonsäureethylester

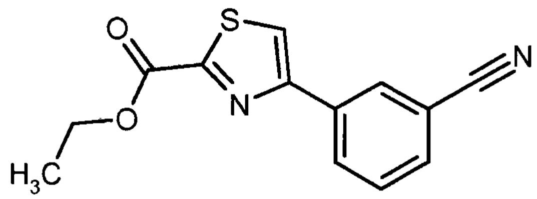

5.00 g (34.4 mmol) 3-Acetylbenzolcarbonitril in 40 ml konz. Essigsäure werden bei Raumtemperatur mit 10 Tropfen einer konz. wässrigen Hydrogenchlorid-Lösung versetzt. Anschließend werden 1.8 ml (34.4 mmol) Brom in 10 ml konz. Essigsäure innerhalb 1 h zugetropft und nach erfolgerter Zugabe wird die Reaktionsmischung auf Eis gegossen. Nach Extraktion der wässrigen Phase mit Dichlormethan werden die vereinigten organischen Phasen über $MgSO_4$ getrocknet, filtriert und i. Vak. eingeengt. Der erhaltene Feststoff (8.00 g) wird in 250 ml EtOH vorgelegt, unter Rückfluss erhitzt und tropfenweise mit einer Lösung aus 3.77 g (28.3 mmol) Ethylamino(thioxo)acetat in 50 ml Ethanol versetzt und 3 h unter Rückfluss gerührt. Die Reaktionslösung wird abgekühlt und der entstandene Niederschlag abfiltriert. Die Mutterlauge wird i. Vak. eingeengt und der Rückstand in wenig Ethanol aufgenommen und anschließend der entstandene Feststoff abfiltriert. Man erhält nach Vereinigung der Feststoffe 5.75 g (65% d. Th.) der Titelverbindung.
$^1$H-NMR (400 MHz, DMSO-$d_6$): $\delta$ 8.74 (s, 1H), 8.45 (s, 1H), 8.35 (d, 1H), 7.88 (d, 1H), 7.77-7.62 (m, 1H), 4.43 (q, 2H), 1.37 (t, 3H).
LC-MS (Methode 5): $R_t$ = 1.15 min; MS (ESIpos): m/z = 259 $[M+H]^+$.

**Beispiel 35A**

5-Brom-4-(3-cyanphenyl)-1,3-thiazol-2-carbonsäureethylester

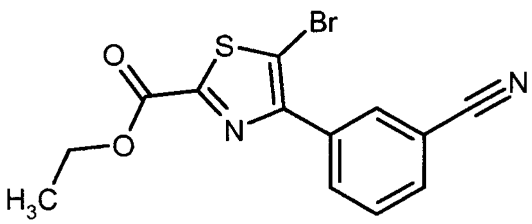

1.03 g (3.98 mmol) der Verbindung aus Beispiel 34A in 50 ml konz. Essigsäure werden bei Raumtemperatur mit 3.18 g (19.9 mmol) Brom und 1.95 g (19.9 mmol) Kaliumacetat versetzt. Es wird für 12 h bei 100°C gerührt, wobei nach 6 und 9 h nochmals gleiche Mengen an Brom (jeweils 3.18 g, 19.9 mmol) und Kaliumacetat (jeweils 1.95 g, 19.9 mmol) zugefügt werden. Anschließend wird die Reaktionslösung mit 1 M wässriger Natriumthiosulfat-Lösung versetzt und mit Dichlormethan extrahiert. Die vereinigten organischen Phasen werden über $MgSO_4$ getrocknet, filtriert und i. Vak. eingeengt. Das Rohprodukt wird mittels Flash-Chromatographie (Laufmittel: Cyclohexan/Essigsäureethylester 20:1→10:1) gereinigt. Man erhält 1.15 g (86% d. Th.) der Titelverbindung.
$^1$H-NMR (400 MHz, DMSO-$d_6$): δ = 8.27 (s, 1H), 8.20 (d, 1H), 7.98 (d, 1H), 7.80-7.74 (m, 1H), 4.42 (q, 2H), 1.35 (t, 3H).
LC-MS (Methode 7): $R_t$ = 2.14 min; MS (ESIpos): m/z = 339 $[M+H]^+$.

**Beispiel 36A**

5-(3-Chlorphenyl)-4-(3-cyanphenyl)-1,3-thiazol-2-carbonsäure

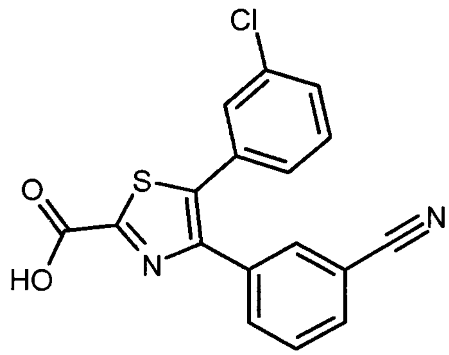

300 mg (0.890 mmol) der Verbindung aus Beispiel 35A und 209 mg (1.34 mmol) (3-Chlorphenyl)boronsäure in 12 ml DME werden bei Raumtemperatur mit 2.2 ml wässriger 2 M Natriumcarbonat-Lösung und 30.8 mg (0.027 mmol) Tetrakis(triphenylphosphin)palladium versetzt und anschließend über Nacht bei 80°C gerührt. Das Rohprodukt wird über eine kurze Kieselgur-Säule filtriert und das Filtrat i. Vak. eingeengt. Der Rückstand wird über präparative HPLC (RP18-Säule; Laufmittel: Acetonitril/Wasser-Gradient) gereinigt. Man erhält 132 mg (37% d. Th.) der Titelverbindung mit 85%iger Reinheit.
$^1$H-NMR (400 MHz, DMSO-$d_6$): δ 7.89 (s, 1H), 7.80 (d, 1H), 7.67 (d, 1H), 7.59-7.52 (m, 1H), 7.52-7.47 (m, 1H), 7.47-7.41 (m, 2H), 7.34-7.29 (m, 1H).
LC-MS (Methode 1): $R_t$ = 2.95 min; MS (ESIpos): m/z = 341 $[M+H]^+$.

**Beispiel 37A**

5-(3-Chlor-5-fluorphenyl)-4-(3-cyanphenyl)-1,3-thiazol-2-carbonsäure

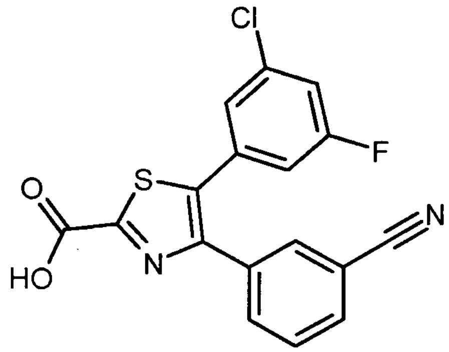

300 mg (0.890 mmol) der Verbindung aus Beispiel 35A und 233 mg (1.34 mmol) (3-Chlor-5-fluorphenyl)boronsäure in 12 ml DME werden bei Raumtemperatur mit 2.2 ml wässriger 2 M Natriumcarbonat-Lösung und 30.8 mg (0.027 mmol) Tetrakis(triphenylphosphin)palladium versetzt und anschließend über Nacht bei 80°C gerührt. Das Rohprodukt wird über eine kurze Kieselgur-Säule filtriert und das Filtrat i. Vak. eingeengt. Der Rückstand wird über präparative HPLC (RP18-Säule; Laufmittel: Acetonitril/Wasser-Gradient) gereinigt. Man erhält 116 mg (36% d. Th.) der Titelverbindung.
$^1$H-NMR (400 MHz, DMSO-$d_6$): $\delta$ = 7.94 (s, 1H), 7.80 (d, 1H), 7.64 (d, 1H), 7.57 - 7.52 (m, 1H), 7.50 (dt, 1H), 7.28 - 7.25 (m, 1H), 7.22 (dt, 1H).
LC-MS (Methode 1): $R_t$ = 3.05 min; MS (ESIpos): m/z = 359 $[M+H]^+$.

**Beispiel 38A**

4,5-Bis(3-cyanphenyl)-1,3-thiazol-2-carbonsäure

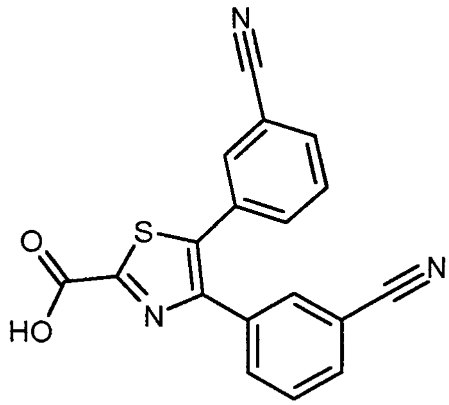

400 mg (1.19 mmol) der Verbindung aus Beispiel 35A und 261 mg (1.78 mmol) (3-Cyanphenyl)boronsäure in 15 ml DME werden bei Raumtemperatur mit 3.0 ml wässriger 2 M Natriumcarbonat-Lösung und 41.1 mg (0.036 mmol) Tetrakis(triphenylphosphin)palladium versetzt und anschließend über Nacht bei 80°C gerührt. Das Rohprodukt wird über eine kurze Kieselgur-Säule filtriert und das Filtrat i. Vak. eingeengt. Der Rückstand wird mittels Flash-Chromatographie (Laufmittel: Dichlormethan) gereinigt. Man erhält 104 mg (26% d. Th.) der Titelverbindung.
$^1$H-NMR (400 MHz, DMSO-$d_6$): $\delta$ = 7.90-7.77 (m, 4H), 7.67-7.50 (m, 4H).
LC-MS (Methode 5): $R_t$ = 0.91 min; MS (ESIpos): m/z = 332 $[M+H]^+$.

**Beispiel 39A**

4,5-Bis(3-chlorphenyl)-1,3-thiazol-2-carbonsäure

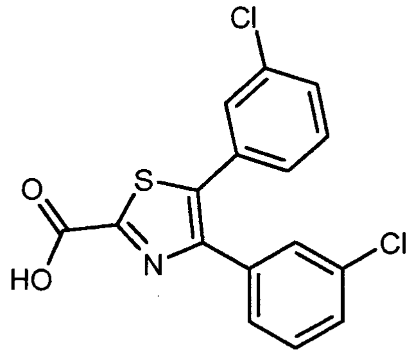

1.00 g (2.88 mmol) der Verbindung aus Beispiel 5A und 677 mg (4.33 mmol) (3-Chlorphenyl)boronsäure in 40 ml DME werden bei Raumtemperatur mit 7.2 ml wässriger 2 M Natriumcarbonat-Lösung und 167 mg (0.144 mmol) Tetrakis(triphenylphosphin)palladium versetzt und anschließend über Nacht bei 80°C gerührt. Die Reaktionsmischung wird mit gesättigter wässriger AmmoniumchloridLösung versetzt und anschließend mit Dichlormethan extrahiert. Die organische Phase wird über Magnesiumsulfat getrocknet, filtriert und i. Vak. eingeengt. Der Rückstand wird über präparative HPLC (RP18-Säule; Laufmittel: Acetonitril/Wasser-Gradient) gereinigt. Man erhält 173 mg (17% d. Th.) der Titelverbindung.
$^1$H-NMR (400 MHz, DMSO-$d_6$): $\delta$ = 7.51 (s, 1H), 7.49-7.30 (m, 5H), 7.30-7.22 (m, 2H)
LC-MS (Methode 5): $R_t$ = 1.29 min; MS (ESIpos): m/z = 350 $[M+H]^+$.

**Beispiel 40A**

4-(3-Chlorphenyl)-5-(3-cyan-5-fluorphenyl)-1, 3-thiazol-2-carbonsäure

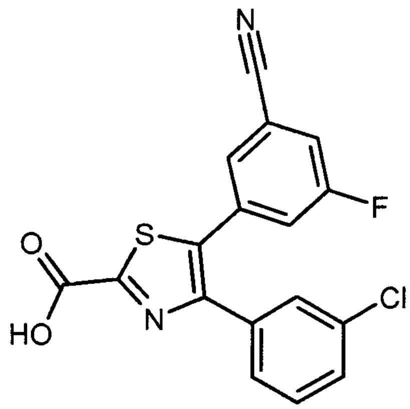

1.00 g (2.89 mmol) der Verbindung aus Beispiel 5A und 714 mg (4.33 mmol) der Boronsäure aus Beispiel 22A in 40 ml DME werden bei Raumtemperatur mit 7.2 ml wässriger 2 M Natriumcarbonat-Lösung und 167 mg (0.144 mmol) Tetrakis(triphenylphosphin)palladium versetzt und anschließend für 24 h bei 80°C gerührt. Die Reaktionsmischung wird mit gesättigter wässriger AmmoniumchloridLösung versetzt und anschließend mit Dichlormethan extrahiert. Die organische Phase wird über Magnesiumsulfat getrocknet, filtriert und i. Vak. eingeengt. Der Rückstand wird über präparative HPLC (RP18-Säule; Laufmittel: Acetonitril/Wasser-Gradient) gereinigt. Man erhält 606 mg (53% d. Th.) der Titelverbindung mit 90%iger Reinheit.
LC-MS (Methode 5): $R_t$ = 1.10 min; MS (ESIpos): m/z = 359 $[M+H]^+$.

**Ausführungsbeispiele:**

**Beispiel 1**

1-{[4-(3-Chlor-4-fluorphenyl)-5-(3-chlor-5-fluorphenyl)-1,3-thiazol-2-yl]carbonyl}imidazolidin-4-on

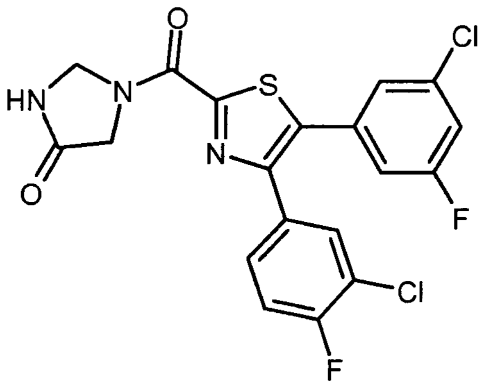

93.0 mg (0.24 mmol) der Verbindung aus Beispiel 8A, 53.0 mg (0.27 mmol) der Verbindung aus Beispiel 20A und 188 mg (0.36 mmol) PyBOP werden in 3 ml Tetrahydrofuran vorgelegt und bei Raumtemperatur mit 88 µl (0.51 mmol) N,N-Diisopropylethylamin versetzt. Man rührt bei Raumtemperatur über Nacht und reinigt anschließend die Reaktionsmischung über präparative HPLC (RP18-Säule; Laufmittel: Acetonitril/Wasser-Gradient). Man erhält 66.0 mg (60% d. Th.) der Titelverbindung.
$^1$H-NMR (400 MHz, DMSO-$d_6$): $\delta$ = 8.81 (s, 0.6H), 8.72 (s, 0.4H), 7.76-7.71 (m, 1H), 7.60 (dt, 1H), 7.50-7.33 (m, 4H), 5.49 (s, 0.8H), 4.94 (s, 1.2H), 4.59 (s, 1.2H), 4.03 (s, 0.8H).
LC-MS (Methode 1): $R_t$ = 2.65 min; MS (ESIpos): m/z = 454 $[M+H]^+$.

**Beispiel 2**

1-{[4-(3-Chlor-4-fluorphenyl)-5-(3-chlorphenyl)-1,3-thiazol-2-yl]carbonyl}imidazolidin-4-on

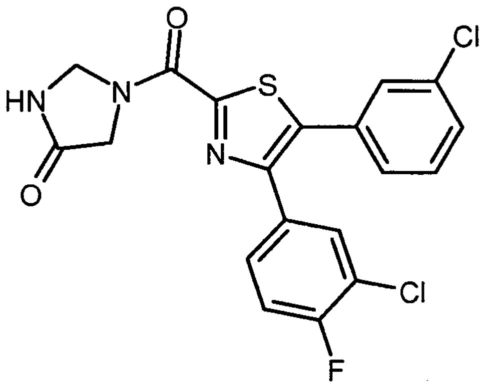

80.0 mg (0.22 mmol) der Verbindung aus Beispiel 9A, 47.8 mg (0.24 mmol) der Verbindung aus Beispiel 20A und 170 mg (0.33 mmol) PyBOP werden in 3.2 ml Tetrahydrofuran -vorgelegt und bei Raumtemperatur mit 79 µl (0.46 mmol) N,N-Diisopropylethylamin versetzt. Man rührt bei Raumtemperatur über Nacht. Anschließend nimmt man das Reaktionsgemisch in Wasser auf, engt ein, saugt den Niederschlag ab und erhält 40.0 mg (42% d. Th.) der Titelverbindung.
$^1$H-NMR (400 MHz, DMSO-$d_6$): $\delta$ = 8.80 (s, 0.6H), 8.71 (s, 0.4H), 7.70 (d, 1H), 7.58-7.54 (m, 2H), 7.52-7.36 (m, 4H), 5.50 (s, 0.8H), 4.94 (s, 1.2H), 4.60 (s, 1.2H), 4.03 (s, 0.8H).
LC-MS (Methode 1): $R_t$ = 2.63 min; MS (ESIpos): m/z = 436 $[M+H]^+$.

**Beispiel 3**

1-{[5-(3-Chlor-5-fluorphenyl)-4-(3-chlorphenyl)-1,3-thiazol-2-yl]carbonyl}imidazolidin-4-on

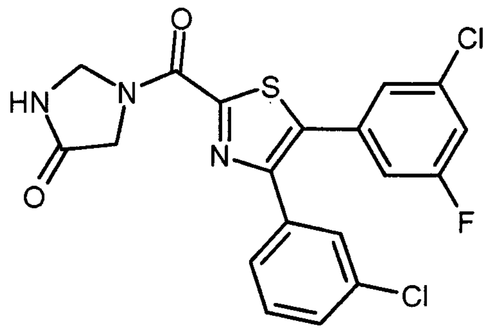

Die Herstellung der Titelverbindung erfolgt ausgehend von der Verbindung aus Beispiel 10A analog zur Synthese der Verbindung aus Beispiel 1. Man erhält 40.0 mg (41% d. Th.) der Titelverbindung.

$^1$H-NMR (400 MHz, DMSO-$d_6$): $\delta$ = 8.81 (s, 0.6H), 8.71 (s, 0.4H), 7.63-7.56 (m, 2H), 7.50-7.45 (m, 1H), 7.45-7.32 (m, 4H), 5.49 (s, 0.8H), 4.94 (s, 1.2H), 4.59 (s, 1.2H), 4.03 (s, 0.8H).

LC-MS (Methode 7): $R_t$ = 2.19 min; MS (ESIpos): m/z = 436 $[M+H]^+$.

**Beispiel 4**

1-{[5-(3-Chlor-4-fluorphenyl)-4-(3-chlorphenyl)-1,3-thiazol-2-yl]carbonyl}imidazolidin-4-on

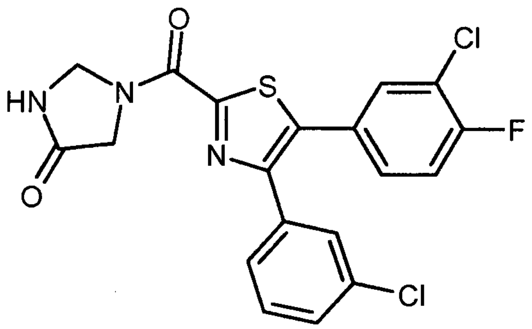

59.0 mg (0.16 mmol) der Verbindung aus Beispiel 11A, 35.3 mg (0.18 mmol) der Verbindung aus Beispiel 20A und 125 mg (0.24 mmol) PyBOP werden in 3 ml Tetrahydrofuran vorgelegt und bei Raumtemperatur mit 0.06 ml (0.34 mmol) N,N-Diisopropylethylamin versetzt. Man rührt bei Raumtemperatur über Nacht. Anschließend nimmt man das Reaktionsgemisch in Wasser auf, engt ein, saugt den Niederschlag ab, kristallisiert den Niederschlag aus Essigsäureethylester um und erhält 20.0 mg (29% d. Th.) der Titelverbindung.

$^1$H-NMR (400 MHz, DMSO-$d_6$): $\delta$ = 8.81 (s, 0.6H), 8.71 (s, 0.4H), 7.73 (dt, 1H), 7.59-7.49 (m, 2H), 7.48-7.33 (m, 4H), 5.50 (s, 0.8H), 4.94 (s, 1.2H), 4.59 (s, 1.2H), 4.03 (s, 0.8H).

LC-MS (Methode 1): $R_t$ = 2.60 min; MS (ESIpos): m/z = 436 $[M+H]^+$.

**Beispiel 5**

5-{4-(3-Chlorphenyl)-2-[(4-oxoimidazolidin-1-yl)carbonyl]-1,3-thiazol-5-yl}-2-fluorbenzolcarbonitril

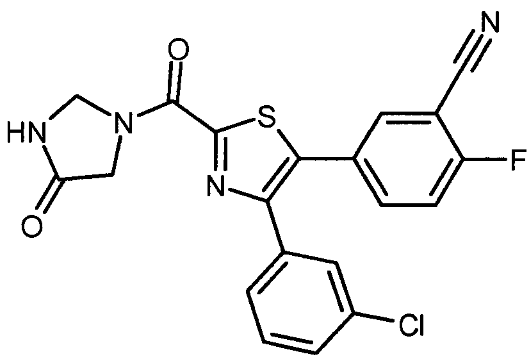

42.4 mg (0.12 mmol) der Verbindung aus Beispiel 12A, 26.0 mg (0.13 mmol) der Verbindung aus Beispiel 20A und 92.3 mg (0.18 mmol) PyBOP werden in 2 ml Tetrahydrofuran vorgelegt und bei Raumtemperatur mit 66 µl (0.38 mmol) N,N-Diisopropylethylamin versetzt. Man rührt bei Raumtemperatur über Nacht und reinigt anschließend die Reaktionsmischung über präparative HPLC (RP18-Säule; Laufmittel: Acetonitril/Wasser-Gradient unter Zusatz von 0.1% Ameisensäure). Man erhält 33.7 mg (65% d. Th.) der Titelverbindung.
$^1$H-NMR (400 MHz, DMSO-$d_6$): $\delta$ = 8.81 (s, 0.6H), 8.71 (s, 0.4H), 8.14 (dt, 1H), 7.79 (ddd, 1H), 7.66-7.57 (m, 2H), 7.49-7.29 (m, 3H), 5.50 (s, 0.8H), 4.94 (s, 1.2H), 4.60 (s, 1.2H), 4.03 (s, 0.8H).
LC-MS (Methode 7): $R_t$ = 1.88 min; MS (ESIpos): m/z = 427 $[M+H]^+$.

**Beispiel 6**

1-{[4-(3-Chlor-5-fluorphenyl)-5-(3-chlorphenyl)-1,3-thiazol-2-yl]carbonyl}imidazolidin-4-on

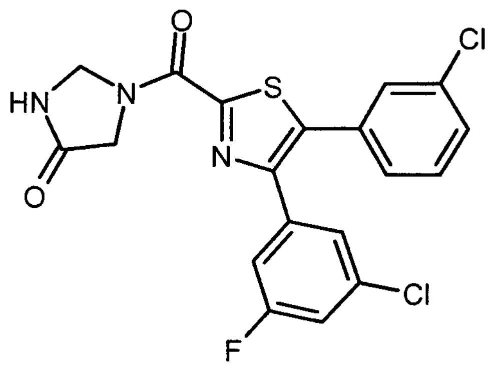

Die Herstellung der Titelverbindung erfolgt ausgehend von der Verbindung aus Beispiel 11A analog zur Synthese der Verbindung aus Beispiel 4. Man erhält 4.5 mg (19% d. Th.) der Titelverbindung.
$^1$H-NMR (400 MHz, DMSO-$d_6$): $\delta$ = 8.81 (s, 0.6H), 8.70 (s, 0.4H), 7.61-7.56 (m, 2H), 7.54-7.47 (m, 2H), 7.42-7.38 (m, 1H), 7.34 (dt, 1H), 7.28 (tt, 1H), 5.50 (s, 0.8H), 4.94 (s, 1.2H), 4.61 (s, 1.2H), 4.03 (s, 0.8H).
LC-MS (Methode 7): $R_t$ = 2.15 min; MS (ESIpos): m/z = 436 $[M+H]^+$.

**Beispiel 7**

3-{4-(3-Chlorphenyl)-2-[(4-oxoimidazolidin-1-yl)carbonyl]-1,3-thiazol-5-yl}benzolcarbonitril

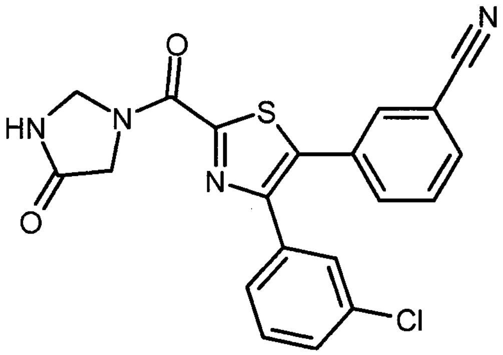

Die Herstellung der Titelverbindung erfolgt ausgehend von der Verbindung aus Beispiel 7A analog zur Synthese der Verbindung aus Beispiel 5. Man erhält 12.0 mg (22% d. Th.) der Titelverbindung.
$^1$H-NMR (400 MHz, DMSO-$d_6$): $\delta$ = 8.81 (s, 0.6H), 8.71 (s, 0.4H), 8.01-7.92 (m, 2H), 7.75-7.71 (m, 1H), 7.66 (t, 1H), 7.56-7.53 (m, 1H), 7.49-7.37 (m, 2H), 7.34 (tt, 1H), 5.50 (s, 0.8H), 4.95 (s, 1.2H), 4.60 (s, 1.2H), 4.03 (s, 0.8H).
LC-MS (Methode 5): $R_t$ = 1.16 min; MS (ESIpos): m/z = 409 $[M+H]^+$.

**Beispiel 8**

1-({4-(3-Chlorphenyl)-5-[3-(trifluormethyl)phenyl]-1,3-thiazol-2-yl}carbonyl)imidazolidin-4-on

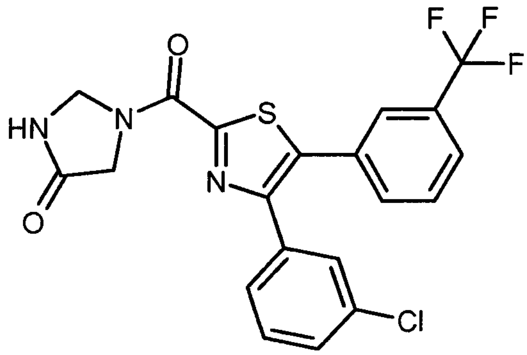

100 mg (0.17 mmol) der Verbindung aus Beispiel 14A, 15.8 mg (0.18 mmol) 4-Imidazolidinon und 95.3 mg (0.18 mmol) PyBOP werden in 1.2 ml Tetrahydrofuran vorgelegt und bei Raumtemperatur mit 32 µl (0.18 mmol) N,N-Diisopropylethylamin versetzt. Man rührt bei Raumtemperatur über Nacht und reinigt anschließend die Reaktionsmischung über präparative HPLC (RP18-Säule; Laufmittel: Acetonitril/Wasser-Gradient) und zusätzlich präparative Dünnschichtchromatographie (Kieselgel; Laufmittel Dichlormethan/Methanol 10/1). Man erhält 34.8 mg (42% d. Th.) der Titelverbindung.
$^1$H-NMR (400 MHz, DMSO-$d_6$): $\delta$ = 8.81 (s, 0.6H), 8.71 (s, 0.4H), 7.85 (d, 1H), 7.78-7.68 (m, 3H), 7.54-7.51 (m, 1H), 7.49-7.34 (m, 3H), 5.50 (s, 0.8H), 4.95 (s, 1.2H), 4.60 (s, 1.2H), 4.04 (s, 0.8H).
LC-MS (Methode 5): $R_t$ = 1.34 min; MS (ESIpos): m/z = 452 $[M+H]^+$.

**Beispiel 9**

5-{4-(3-Chlor-4-fluorphenyl)-2-[(4-oxoimidazolidin-1-yl)carbonyl]-1,3-thiazol-5-yl}-2-fluorbenzolcarbonitril

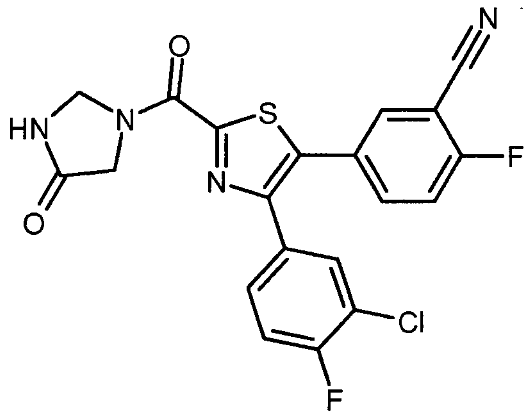

Die Herstellung der Titelverbindung erfolgt ausgehend von der Verbindung aus Beispiel 15A analog zur Synthese der Verbindung aus Beispiel 5. Man erhält 17.3 mg (35% d. Th.) der Titelverbindung.
$^1$H-NMR (400 MHz, DMSO-$d_6$): δ = 8.81 (s, 0.6H), 8.72 (s, 0.4H), 8.16 (dt, 1H), 7.82-7.73 (m, 2H), 7.62 (t, 1H), 7.46-7.33 (m, 2H), 5.50 (s, 0.8H), 4.94 (s, 1.2H), 4.60 (s, 1.2H), 4.03 (s, 0.8H).
LC-MS (Methode 7): $R_t$ = 1.91 min; MS (ESIpos): m/z = 445 $[M+H]^+$.

**Beispiel 10**

3-{4-(3-Chlor-4-fluorphenyl)-2-[(4-oxoimidazolidin-1-yl)carbonyl]-1,3-thiazol-5-yl}benzolcarbonitril

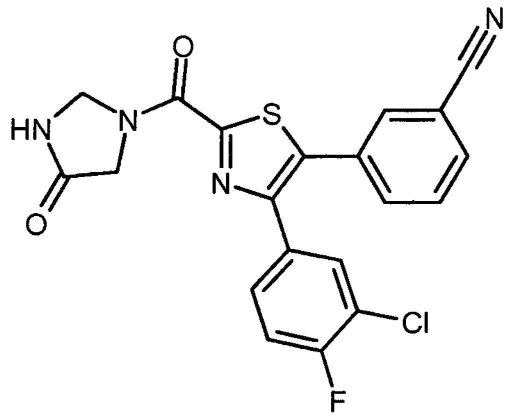

Die Herstellung der Titelverbindung erfolgt ausgehend von der Verbindung aus Beispiel 16A analog zur Synthese der Verbindung aus Beispiel 5. Man erhält 9.3 mg (15% d. Th.) der Titelverbindung.
$^1$H-NMR (400 MHz, DMSO-$d_6$): δ = 8.81 (s, 0.6H), 8.72 (s, 0.4H), 8.03-8.00 (m, 1H), 7.95 (d, 1H), 7.75-7.63 (m, 3H), 7.48-7.34 (m, 2H), 5.50 (s, 0.8H), 4.94 (s, 1.2H), 4.60 (s, 1.2H), 4.04 (s, 0.8H).
LC-MS (Methode 7): $R_t$ = 1.85 min; MS (ESIpos): m/z = 427 $[M+H]^+$.

**Beispiel 11**

3-{4-(3-Chlor-4-fluorphenyl)-2-[(4-oxoimidazolidin-1-yl)carbonyl]-1,3-thiazol-5-yl}-5-fluorbenzolcarbonitril

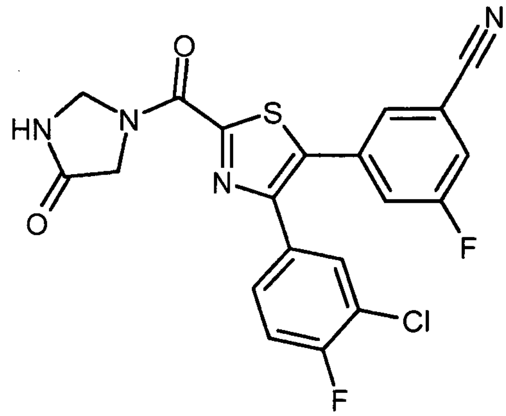

108 mg (0.25 mmol) der Verbindung aus Beispiel 7A und 61.9 mg (0.38 mmol) der Verbindung aus Beispiel 22A werden unter Argon in 3.5 ml 1,2-Dimethoxyethan vorgelegt und mit 1.3 ml (1.25 mmol) wässriger Natriumhydrogencarbonat-Lösung (10%ig) und 8.7 mg (8 μmol) Tetrakis(triphenylphosphin)palladium(0) versetzt. Es wird bei 80°C über Nacht gerührt. Man engt ein, reinigt den Rückstand über präparative HPLC (RP18-Säule; Laufmittel: Acetonitril/Wasser-Gradient) und zusätzlich präparative Dünnschichtchromatographie (Kieselgel; Laufmittel Essigsäureethylester/Cyclohexan 1/1) und erhält 4.3 mg (4% d. Th.) der Titelverbindung.

$^1$H-NMR (400 MHz, DMSO-$d_6$): δ = 8.81 (s, 0.6H), 8.72 (s, 0.4H), 8.01 (d, 1H), 7.88-7.85 (m, 1H), 7.77-7.71 (m, 2H), 7.48-7.33 (m, 2H), 5.50 (s, 0.8H), 4.94 (s, 1.2H), 4.60 (s, 1.2H), 4.04 (s, 0.8H).

LC-MS (Methode 7): $R_t$ = 1.91 min; MS (ESIpos): m/z = 445 [M+H]$^+$.

**Beispiel 12**

3-{5-(3-Chlor-5-fluorphenyl)-2-[(4-oxoimidazolidin-1-yl)carbonyl]-1,3-thiazol-4-yl}-5-fluorbenzolcarbonitril

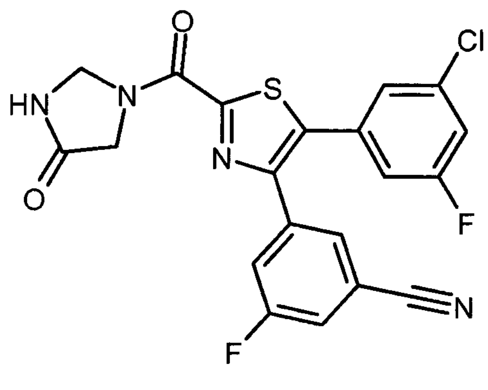

65.0 mg (0.143 mmol) der Verbindung aus Beispiel 26A mit 83%iger Reinheit, 31.5 mg (0.158 mmol) der Verbindung aus Beispiel 20A und 112 mg (0.215 mmol) PyBOP werden in 6.0 ml Tetrahydrofuran vorgelegt und bei Raumtemperatur mit 80 μl (0.458 mmol) *N,N*-Diisopropylethylamin versetzt. Man rührt bei Raumtemperatur über Nacht, verdünnt die Reaktionslösung mit Dichlormethan und wäscht anschließend mit Wasser. Nach Trocknung der organischen Phase über Magnesiumsulfat, Filtration und Einengen i. Vak. wird das Rohprodukt in wenig Acetonitril aufgenommen und anschließend der entstandene Feststoff abfiltriert. Man erhält 30.0 mg (47% d. Th.) der Titelverbindung.

$^1$H-NMR (400 MHz, DMSO-$d_6$): δ = 8.81 (s, 0.6H), 8.71 (s, 0.4H), 7.93 (d, 1H), 7.82 (d, 1H), 7.74-7.57 (m, 2H), 7.50-7.31 (m, 2H), 5.51 (s, 0.8H), 4.95 (s, 1.2H), 4.63 (s, 1.2H), 4.04 (s, 0.8H).

LC-MS (Methode 5): $R_t$ = 1.25 min; MS (ESIpos): m/z = 445 [M+H]$^+$.

**Beispiel 13**

5-{5-(3-Chlor-5-fluorphenyl)-2-[(4-oxoimidazolidin-1-yl)carbonyl]-1,3-thiazol-4-yl}-2-fluorbenzolcarbonitril

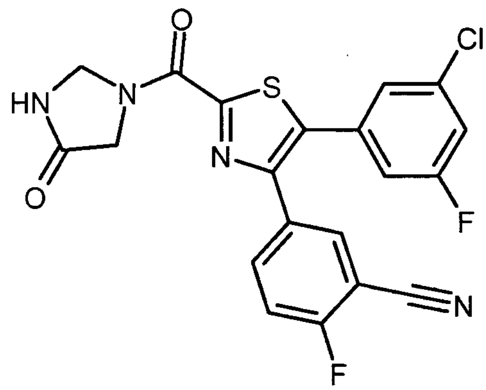

45.0 mg (0.119 mmol) der Verbindung aus Beispiel 30A mit 84%iger Reinheit, 26.3 mg (0.131 mmol) der Verbindung aus Beispiel 20A und 93.2 mg (0.179 mmol) PyBOP werden in 4.8 ml Tetrahydrofuran vorgelegt und bei Raumtemperatur mit 67 µl (0.382 mmol) *N*,*N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Man erhält 25.0 mg (56% d. Th.) der Titelverbindung.

$^1$H-NMR (400 MHz, DMSO-$d_6$): $\delta$ = 8.80 (s, 0.6H), 8.73 (s, 0.4H), 8.20-8.09 (m, 1H), 7.77 (m, 1H), 7.67-7.50 (m, 2H), 7.41 (s, 1H), 7.36 (d, 1H), 5.50 (s, 0.8H), 4.94 (s, 1.2H), 4.62 (s, 1.2 H), 4.04 (s, 0.8H).

LC-MS (Methode 5): $R_t$ = 1.26 min; MS (ESIpos): m/z = 445 $[M+H]^+$.

**Beispiel 14**

5-{5-(3-Chlorphenyl)-2-[(4-oxoimidazolidin-1-yl)carbonyl]-1,3-thiazol-4-yl}-2-fluorbenzolcarbonitril

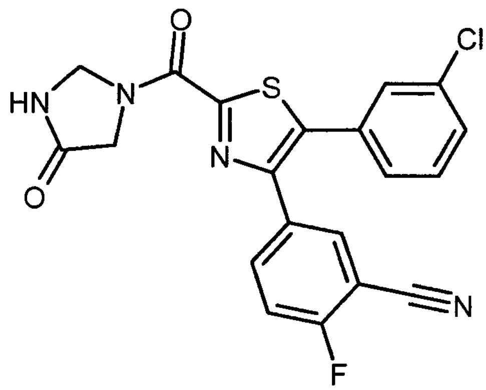

80.0 mg (0.223 mmol) der Verbindung aus Beispiel 31A, 49.1 mg (0.245 mmol) der Verbindung aus Beispiel 20A und 174 mg (0.334 mmol) PyBOP werden in 7.0 ml Tetrahydrofuran vorgelegt und bei Raumtemperatur mit 124 µl (0.714 mmol) *N*,*N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Man erhält 42.5 mg (45% d. Th.) der Titelverbindung.

$^1$H-NMR (400 MHz, DMSO-$d_6$): $\delta$ = 8.80 (s, 0.6H), 8.73 (s, 0.4H), 8.17-8.05 (m, 1H), 7.85-7.68 (m, 1 H), 7.60-7.52 (m,

3H), 7.48 (t, 1H), 7.37 (d, 1H), 5.51 (s, 0.8H), 4.94 (s, 1.2H), 4.62 (s, 1.2H), 4.04 (s, 0.8H).
LC-MS (Methode 1): $R_t$ = 2.32 min; MS (ESIpos): m/z = 427 $[M+H]^+$.

**Beispiel 15**

5-{5-(3-Cyan-5-fluorphenyl)-2-[(4-oxoimidazolidin-1-yl)carbonyl]-1,3-thiazol-4-yl}-2-fluorbenzolcarbonitril

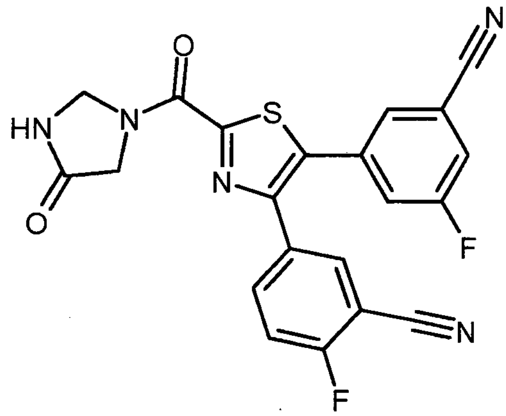

45.0 mg der Verbindung aus Beispiel 32A mit 61%iger Reinheit (0.075 mmol), 16.5 mg (0.082 mmol) der Verbindung aus Beispiel 20A und 58.3 mg (0.112 mmol) PyBOP werden in 3 ml Tetrahydrofuran vorgelegt und bei Raumtemperatur mit 42 µl (0.239 mmol) *N,N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt mit Acetonitril versetzt und anschließend der entstandene Feststoff abfiltriert. Man erhält 17.4 mg (53% d. Th.) der Titelverbindung.
$^1$H-NMR (400 MHz, DMSO-$d_6$): δ = 8.81 (s, 0.6H), 8.74 (s, 0.4H), 8.22-8.11 (m, 1H), 8.01 (d, 1H), 7.86 (s, 1H), 7.82-7.68 (m, 2H), 7.54 (td, 1H), 5.51 (s, 0.8H), 4.95 (s, 1.2H), 4.62 (s, 1.2H), 4.04 (s, 0.8H).
LC-MS (Methode 5): $R_t$ = 1.11 min; MS (ESIpos): m/z = 436 $[M+H]^+$.

**Beispiel 16**

5-{5-(3-Cyanphenyl)-2-[(4-oxoimidazolidin-1-yl)carbonyl]-1,3-thiazol-4-yl}-2-fluorbenzolcarbonitril

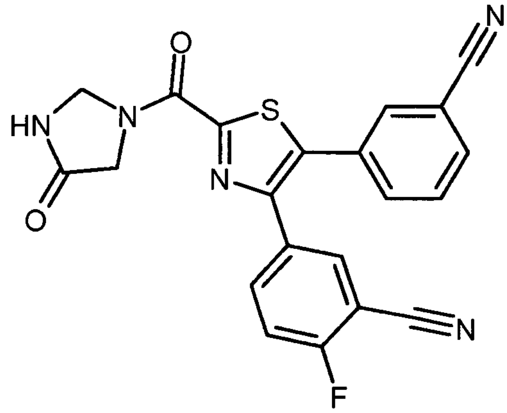

45.0 mg (0.129 mmol) der Verbindung aus Beispiel 33A, 28.4 mg (0.142 mmol) der Verbindung aus Beispiel 20A und 101 mg (0.193 mmol) PyBOP werden in 4.0 ml Tetrahydrofuran vorgelegt und bei Raumtemperatur mit 72 µl (0.412 mmol) *N,N*-Diisopropylethylamin versetzt. Man rührt bei Raumtemperatur über Nacht, verdünnt die Reaktionslösung

mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt in wenig Acetonitril aufgenommen und anschließend der entstandene Feststoff abfiltriert. Das Rohprodukt wird über präparative HPLC (RP18-Säule; Laufmittel: Acetonitril/Wasser-Gradient) gereinigt. Man erhält 15.2 mg (28% d. Th.) der Titelverbindung.
$^1$H-NMR (400 MHz, DMSO-$d_6$): $\delta$ = 8.81 (s, 0.6H), 8.73 (s, 0.4H), 8.14-8.09 (m, 1H), 8.02 (d, 1H), 7.96 (d, 1H), 7.80-7.69 (m, 2H), 7.68-7.62 (m, 1H), 7.58-7.50 (m, 1H), 5.51 (s, 0.8H), 4.95 (s, 1.2 H), 4.62 (s, 1.2H), 4.04 (s, 0.8H).
LC-MS (Methode 7): $R_t$ = 1.68 min; MS (ESIpos): m/z = 418 $[M+H]^+$.

**Beispiel 17**

3-{5-(3-Chlorphenyl)-2-[(4-oxoimidazolidin-1-yl)carbonyl]-1,3-thiazol-4-yl}benzolcarbonitril

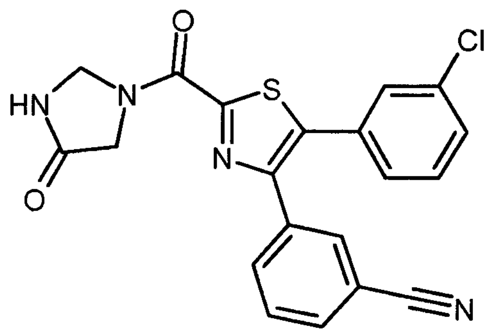

80.0 mg der Verbindung aus Beispiel 36A mit 85%iger Reinheit (0.235 mmol), 51.7 mg (0.258 mmol) der Verbindung aus Beispiel 20A und 183 mg (0.352 mmol) PyBOP werden in 4.5 ml Tetrahydrofuran vorgelegt und bei Raumtemperatur mit 131 µl (0.751 mmol) *N*,*N*-Diisopropylethylamin versetzt. Man rührt bei Raumtemperatur über Nacht, verdünnt die Reaktionslösung mit Dichlormethan und wäscht anschließend mit Wasser. Nach Trocknung der organischen Phase über Magnesiumsulfat, Filtration und Eineengen i. Vak. wird das Rohprodukt durch Flash-Chromatographie (Laufmittel: Dichlormethan/Methanol 100:1) vorgereinigt. Anschließend wird das Rohprodukt über präparative HPLC (RP18-Säule; Laufmittel: Acetonitril/Wasser-Gradient) gereinigt und der erhaltene Feststoff in Diethylether/Acetonitril aufgenommen, für 30 min gerührt und anschließend filtriert. Man erhält 60.0 mg (74% d. Th.) der Titelverbindung.
$^1$H-NMR (400 MHz, DMSO-$d_6$): $\delta$ = 8.81 (s, 0.6H), 8.72 (s, 0.4H), 7.98 (s, 1H), 7.86 (d, 1H), 7.79-7.67 (m, 1H), 7.64-7.52 (m, 3H), 7.49 (t, 1H), 7.37 (d, 1H), 5.51 (s, 0.8H), 4.95 (s, 1.2H), 4.62 (s, 1.2H), 4.04 (s, 0.8H).
LC-MS (Methode 7): $R_t$ = 1.90 min; MS (ESIpos): m/z = 409 $[M+H]^+$.

**Beispiel 18**

3-{5-(3-Chlor-5-fluorphenyl)-2-[(4-oxoimidazolidin-1-yl)carbonyl]-1,3-thiazol-4-yl}benzolcarbonitril

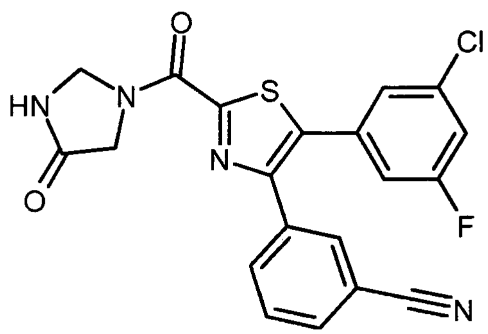

80.0 mg (0.223 mmol) der Verbindung aus Beispiel 37A, 49.1 mg (0.245 mmol) der Verbindung aus Beispiel 20A und 174 mg (0.334 mmol) PyBOP werden in 4.5 ml Tetrahydrofuran vorgelegt und bei Raumtemperatur mit 124 µl (0.714

mmol) *N*,*N*-Diisopropylethylamin versetzt. Man rührt bei Raumtemperatur über Nacht, verdünnt die Reaktionslösung mit Dichlormethan und wäscht anschließend mit Wasser. Nach Trocknung der organischen Phase über Magnesiumsulfat, Filtration und Eineengen i. Vak. wird das Rohprodukt durch Flash-Chromatographie (Laufmittel: Dichlormethan/Methanol 100:1) vorgereinigt. Anschließend wird das Rohprodukt über präparative HPLC (RP18-Säule; Laufmittel: Acetonitril/Wasser-Gradient) gereinigt und der erhaltene Feststoff in Diethylether/Acetonitril aufgenommen, für 30 min gerührt und anschließend filtriert. Man erhält 58.0 mg (61% d. Th.) der Titelverbindung.
$^1$H-NMR (DMSO-$d_6$): δ = 8.81 (s, 0.6H), 8.72 (s, 0.4H), 8.02 (d, 1H), 7.92-7.83 (m, 1H), 7.79-7.67 (m, 1H), 7.65-7.57 (m, 2H), 7.39 (d, 1H), 7.35 (dd, 1H), 5.51 (s, 0.8H), 4.94 (s, 1.2H), 4.62 (s, 1.2H), 4.04 (s, 0.8H).
LC-MS (Methode 7): $R_t$ = 1.93 min; MS (ESIpos): m/z = 427 [M+H]$^+$.

**Beispiel 19**

3,3'-{2-[(4-Oxoimidazolidin-1-yl)carbonyl]-1,3-thiazol-4,5-diyl}dibenzolcarbonitril

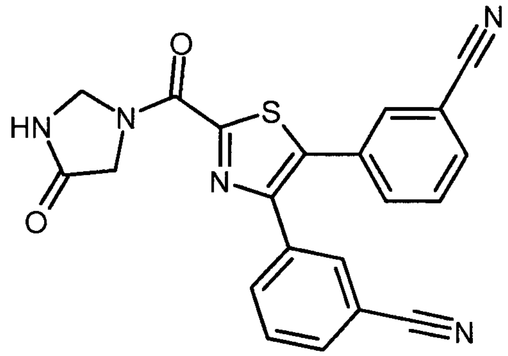

55.0 mg (0.166 mmol) der Verbindung aus Beispiel 38A, 36.5 mg (0.183 mmol) der Verbindung aus Beispiel 20A und 130 mg (0.249 mmol) PyBOP werden in 5.5 ml Tetrahydrofuran vorgelegt und bei Raumtemperatur mit 93 µl (0.531 mmol) *N*,*N*-Diisopropylethylamin versetzt. Man rührt bei Raumtemperatur über Nacht, verdünnt die Reaktionslösung mit Dichlormethan und wäscht anschließend mit Wasser. Nach Trocknung der organischen Phase über Magnesiumsulfat, Filtration und Eineengen i. Vak. wird das Rohprodukt durch Flash-Chromatographie (Laufmittel: Dichlormethan/Methanol 100:1) vorgereinigt. Anschließend wird das Rohprodukt über präparative HPLC (RP18-Säule; Laufmittel: Acetonitril/Wasser-Gradient) gereinigt. Man erhält 37.0 mg (56% d. Th.) der Titelverbindung.
$^1$H-NMR (400 MHz, DMSO-$d_6$): δ = 8.81 (s, 0.6H), 8.72 (s, 0.4H), 8.02-7.98 (m, 2H), 7.96 (d, 1H), 7.87 (d, 1H), 7.76-7.53 (m, 4H), 5.52 (s, 0.8H), 4.95 (s, 1.2H), 4.62 (s, 1.2H), 4.04 (s, 0.8H)
LC-MS (Methode 5): $R_t$ = 1.04 min; MS (ESIpos): m/z = 400 [M+H]$^+$.

**Beispiel 120**

1-{[4,5-Bis(3-chlorphenyl)-1,3-thiazol-2-yl]carbonyl}imidazolidin-4-on

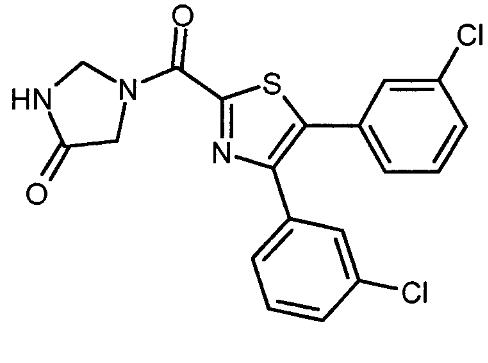

100 mg (0.286 mmol) der Verbindung aus Beispiel 39A, 62.9 mg (0.314 mmol) der Verbindung aus Beispiel 20A und 223 mg (0.428 mmol) PyBOP werden in 4.0 ml Tetrahydrofuran vorgelegt und bei Raumtemperatur mit 159 µl (0.914 mmol) *N*,*N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird der erhaltene Feststoff in Diethylether/Acetonitril aufgenommen, für 30 min gerührt und anschließend filtriert. Man erhält 53.0 mg (44% d. Th.) der Titelverbindung.
$^1$H-NMR (400 MHz, DMSO-$d_6$): δ = 8.81 (s, 0.6H), 8.71 (s, 0.4H), 7.60-7.33 (m, 8H), 5.50 (s, 0.8H), 4.94 (s, 1.2H), 4.60 (s, 1.2H), 4.03 (s, 0.8H).
LC-MS (Methode 12): $R_t$ = 1.18 min; MS (ESIpos): m/z = 418 [M+H]$^+$.

**Beispiel 21**

3-{4-(3-Chlorphenyl)-2-[(4-oxoimidazolidin-1-yl)carbonyl]-1,3-thiazol-5-yl}-5-fluorbenzolcarbonitril

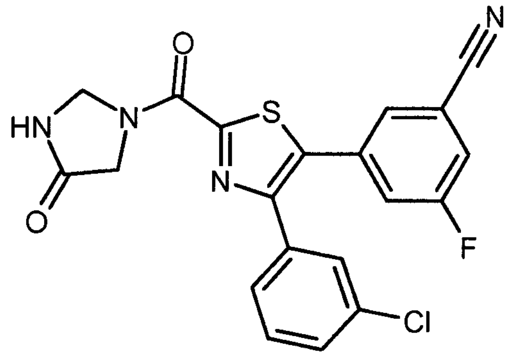

90.0 mg der Verbindung aus Beispiel 40A mit 90%iger Reinheit (0.226 mmol), 49.7 mg (0.248 mmol) der Verbindung aus Beispiel 20A und 176 mg (0.339 mmol) PyBOP werden in 5.0 mal Tetrahydrofuran vorgelegt und bei Raumtemperatur mit 126 µl (0.722 mmol) *N*,*N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Man erhält 62.0 mg (64% d. Th.) der Titelverbindung.
$^1$H-NMR (400 MHz, DMSO-$d_6$): δ = 8.81 (s, 0.6H), 8.71 (s, 0.4H), 8.00 (d, 1H), 7.85 (br. s., 1H), 7.72 (d, 1H), 7.58 (br. s., 1H), 7.53-7.46 (m, 1H), 7.45-7.38 (m, 1H), 7.38-7.30 (m, 1H), 5.50 (s, 0.8H), 4.95 (s, 1.2H), 4.60 (s, 1.2H), 4.03 (s, 0.8H).
LC-MS (Methode 7): $R_t$ = 1.89 min; MS (ESIpos): m/z = 427 [M+H]$^+$.

**B) Bewertung der physiologischen Wirksamkeit**

**Abkürzungen:**

| | |
|---|---|
| DMSO | Dimethylsulfoxid |
| FKS | Fötales Kälber Serum (Biochrom AG, Berlin, Deutschland) |
| PBS | Phosphate buffered saline |
| MTP | Mikrotiterplatte |
| ELISA | Enzyme-linked immunosorbent assay |

Die Eignung der erfindungsgemäßen Verbindungen zur Behandlung von durch Retroviren hervorgerufenen Erkrankungen kann in folgenden Assay-Systemen gezeigt werden:

In vitro Assays

Biochemischer Reverse Transkriptase Assay

Es wird der "Reverse Transcriptase Assay, colorimetric" (Roche Diagnostics GmbH, Mannheim, Deutschland) entsprechend den Herstellerangaben verwendet. Die Prüfsubstanzen werden in DMSO gelöst und in 5er Schritten verdünnt in dem Test eingesetzt (DMSO Endkonzentration 1%). Die resultierenden Werte der photometrischen Auswertung (405/492 nm) sind bei der Negativkontrolle (Ansatz ohne Reverse Transkriptase) kleiner 0,1 und liegen bei der Positivkontrolle (Ansatz ohne Prüfsubstanz) im Bereich von 1,5. Die $IC_{50}$-Werte der Prüfsubstanzen werden als die Konzentration der Prüfsubstanzverdünnung ermittelt, bei der die gemessene optische Dichte 50% der Positivkontrolle beträgt.

Es wird gefunden, dass die erfindungsgemäßen Verbindungen die Reverse Transkriptase Aktivität hemmen. Experimentelle Daten sind in Tabelle A zusammengefasst.

**Light Assay mit wildtyp und Inhibitor-resistenten HI-Reporterviren**

Für diesen Assay werden HIV-$1_{NL4-3}$ Reporterviren verwendet, die anstelle des *nef* Gens das *ht164* Gen (Luziferase 164) tragen. Die Viren werden durch Transfektion von 293T-Zellen mit dem entsprechenden proviralen pNL4-3 Plasmid generiert (Lipofectamine Reagent, Invitrogen, Karlsruhe, Deutschland). Ausgehend von der proviralen Plasmid-DNA werden mit dem "QuikChange 11 XL Site-Directed Mutagenesis Kit" (Stratagene, Cedar Creek, Texas, USA) Viren mit definierten Resistenzmutationen im Reverse Transkriptase Gen hergestellt. Es werden u.a. folgende Mutationen generiert: A98G, A98G-K103N-V108I, A98S, F227C, F227L, G190A, G190S, K101E, K101Q-K103N, K103N, K103N-F227L, K103N-G190A, K103N-G190S, K103N-M230L, K103N-N348I, K103N-P225H, K103N-V108I, K103N-V108I-P225H, K103N-V179F-Y181C, K103N-Y181C, K103N-Y181C-G190A, L100I, L100I-K103N, L100I-K103N-V179I-Y181C, L100I-K103N-Y181C, L234I, N348I, P225H, P236L, V106A, V106A-E138K, V106A-F227C, V106A-F227L, V106I, V106I-Y188L, V106M, V108I, V179F-Y181C, V179I, V1791-Y181C, Y181C, Y181C-G190A, Y181C-M230L, Y181I, Y188L. Mit diesen Reporterviren infizierte MT4 7F2 Zellen sekretieren Luziferase ins Medium, was die luminometrische Quantifizierung der Virusreplikation ermöglicht.

Für den Ansatz einer 96-well MTP werden 3 Millionen MT4 7F2 Zellen pelletiert, in 1 ml RPMI 1640 Medium ohne Phenolrot (Invitrogen, Karlsruhe, Deutschland)/10% FKS/10% AIM-V (Invitrogen, Karlsruhe, Deutschland) suspendiert und zusammen mit einer geeigneten Menge des entsprechenden HIV-$1_{NL4-3}$ Reportervirus für 2 Stunden bei 37°C inkubiert (Pelletinfektion). Nicht adsorbierte Viren werden anschließend mit PBS ausgewaschen, die infizierten Zellen wieder pelletiert und in 8 ml RPMI 1640 Medium ohne Phenolrot/2% oder 10% FKS/10% AIM-V suspendiert. Davon werden 80 µl pro Well in eine weiße 96-well MTP zu 20 µl Prüfsubstanz in geeigneter Verdünnung pipettiert. Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium ohne Phenolrot/2% oder 10% FKS/10% AIM-V. Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden 5 Tage bei 37°C/5% $CO_2$ inkubiert und nach Zugabe von 15 µl Lu164-Substrat (5mg/ml Coelenterazin gelöst in 30 µM Glutathion/DMSO, 100 mM NaCl, 1M MES, 100 mM Glutathion) luminometrisch ausgewertet. Die resultierenden Werte liegen bei der Viruskontrolle im Bereich von 1.000.000 RLUs (relative Lichteinheiten) und bei der Zellkontrolle bei 300 bis 400 RLUs. Die $EC_{50}$-Werte der Prüfsubstanzen werden als die Konzentration ermittelt, bei der die in RLUs gemessene Virusreplikation 50% der unbehandelten infizierten Zellen beträgt.

Es wird gefunden, dass die erfindungsgemäßen Verbindungen die HIV-Replikation hemmen. Experimentelle Daten sind in Tabelle A zusammengefasst.

**PBL- und H9-Assay mit wildtyp HIV-1**

Primäre menschliche Blutlymphozyten (PBLs) werden über Ficoll-Paque Leucosep Röhrchen (Greiner Bio-One, Frickenhausen, Deutschland) aus Blut isoliert und in RPMI 1640 Medium (Invitrogen, Karlsruhe, Deutschland)/10% FKS mit Phytohaemagglutinin (90 µg/ml) und Interleukin-2 (40 U/ml) 3 Tage stimuliert.

Für den Ansatz einer 96-well MTP werden 3 Millionen PBLs pelletiert, in 1 ml RPMI 1640 Medium/10% FKS suspendiert und zusammen mit einer geeigneten Menge HIV-$1_{LAI}$ (NIH AIDS Research & Reference Reagent Program, Germantown, USA) für 2 Stunden bei 37°C inkubiert (Pelletinfektion). Nicht adsorbierte Viren werden anschließend mit PBS ausgewaschen, die infizierten Zellen wieder pelletiert und in 18 ml RPMI 1640 Medium/10% FKS/Interleukin-2 (40 U/ml) suspendiert. Davon werden 180 µl pro well in eine weiße 96-well MTP zu 20 µl Prüfsubstanz in geeigneter

Verdünnung pipettiert. Alternativ wird das HIV nach Zubereitung der Substanzverdünnungen in der MTP zusammen mit den Zellen zupipettiert und wird nicht mehr ausgewaschen (Überstandsinfektion). Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium/10% FKS/Interleukin-2 (40 U/ml). Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz 1% beträgt. Die Testansätze werden bei 37°C/5% $CO_2$ inkubiert. Nach 5 und 7 Tagen erfolgt die Abnahme von jeweils 50 μl zellfreiem Überstand aus jedem Well zur Bestimmung der enthaltenen p24 Menge mittels p24 ELISA (HIV-1 $p24^{CA}$ Antigen Capture Assay Kit, NCI-Frederick Cancer Research and Development Center, Frederick, USA). Aus den resultierenden Werten der photometrischen Auswertung (450/620nm) werden die $EC_{50}$-Werte der Prüfsubstanzen als die Konzentration ermittelt, bei der die p24 Menge 50% der unbehandelten infizierten Zellen beträgt.

Alternativ werden H9-Zellen (ATCC, Wesel, Deutschland) anstelle von PBLs zum Test der Prüfsubstanzen eingesetzt. H9-Zellen werden nach oben beschriebenem Muster in RPMI 1640 Medium mit 2% oder 10% FKS als HIV-$1_{LAI}$ Überstandsinfektion 5 Tage bei 37°C/5% $CO_2$ inkubiert (20 μl Substanzverdünnung und 80 μl Zellen/Virus pro Well). Anschließend wird je Well 10 μl AlamarBlue (Invitrogen, Karlsruhe, Deutschland) zugegeben und die MTPs werden für 3 Stunden bei 37°C inkubiert, bevor die fluorimetrische Auswertung erfolgt (544/590nm). Die resultierenden Werte liegen bei den unbehandelten nicht infizierten Zellen bei etwa 40.000 und bei den unbehandelten infizierten Zellen bei etwa 7.000. Im niedrigen Konzentrationsbereich werden die $EC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen). Außerdem werden im hohen Konzentrationsbereich die $CC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen).

Es wird gefunden, dass die erfindungsgemäßen Verbindungen die HIV-Replikation hemmen. Experimentelle Daten sind in Tabelle A zusammengefasst.

**Assay zur Bestimmung der zytotoxischen Wirkung der Prüfsubstanzen**

Zur Bestimmung der zytotoxischen Wirkung der Prüfsubstanzen in nicht infizierten Zellen werden die Substanzen in entsprechenden Konzentrationen auf durchsichtige 96-well MTPs pipettiert und mit nicht infizierten Zellen (z.B. H9, PBLs, THP-1, MT4 7F2, CEM, Jurkat) inkubiert (analog zu den oben beschriebenen Assays). Nach 5 Tagen wird zu den Testansätzen je Well 1/10 Volumen AlamarBlue zugegeben und die MTPs werden für 3 Stunden bei 37°C inkubiert. Anschließend erfolgt die fluorimetrische Auswertung (544/590nm). Die resultierenden Werte liegen bei nicht behandelten Zellen je nach Zellart zwischen 20.000 und 40.000. Die $CC_{50}$-Werte der Prüfsubstanzen werden als die Konzentration ermittelt, bei der die Fluoreszenz 50% der unbehandelten Zellen beträgt. Prüfsubstanzen, die im Konzentrationsbereich der Wirkung zytotoxische Befunde zeigen, werden nicht in ihrer antiviralen Wirksamkeit bewertet.

**Tabelle A:**

| Beispiel-Nr. | $IC_{50}$ (nM) RT-Assay | $EC_{50}$(nM) H9 Zellen HIV-$1_{LAI}$ 10% FKS | $EC_{50}$ (nM) MT4 7F2 Zellen HIV-$1_{NL4-3}$ Wt 2% FKS | $EC_{50}$ (nM) MT4 7F2 Zellen HIV-$1_{NL4-3}$ K103N-Y181C 2% FKS |
|---|---|---|---|---|
| Beispiel 1 | 61 | 14 | 2 | 55 |
| Beispiel 2 | 17 | 30 | 4 | 91 |
| Beispiel 3 | 20 | 12 | 0.6 | 60 |
| Beispiel 4 | | 80 | 10 | 143 |
| Beispiel 5 | | 80 | 38 | 228 |
| Beispiel 6 | | 95 | 58 | 282 |
| Beispiel 7 | | 20 | 36 | 303 |
| Beispiel 8 | | | 46 | 142 |
| Beispiel 9 | | 40 | 35 | 118 |
| Beispiel 10 | | 5 | 4 | 70 |

(fortgesetzt)

| Beispiel-Nr. | $IC_{50}$ (nM) RT-Assay | $EC_{50}$(nM) H9 Zellen HIV-$1_{LAI}$ 10% FKS | $EC_{50}$ (nM) MT4 7F2 Zellen HIV-$1_{NL4-3}$ Wt 2% FKS | $EC_{50}$ (nM) MT4 7F2 Zellen HIV-$1_{NL4-3}$ K103N-Y181C 2% FKS |
|---|---|---|---|---|
| Beispiel 11 | 13 | 4 | 5 | 69 |
| Beispiel 12 | | 80 | 29 | 119 |
| Beispiel 13 | | 2 | 0,6 | 43 |
| Beispiel 14 | | 4 | 1 | 49 |
| Beispiel 15 | | 2 | 9 | 87 |
| Beispiel 16 | | 2 | 15 | 123 |
| Beispiel 17 | | 2 | 0,5 | 64 |
| Beispiel 18 | | 1 | 0,4 | 38 |
| Beispiel 19 | | 1 | 2 | 94 |
| Beispiel 20 | 47 | 7 | 1 | 149 |
| Beispiel 21 | | 2 | 2 | 79 |

**In vivo Assay**

**Tiermodell:**

NOD Scid Mäuse, in der Regel 5 - 6 Wochen alt, werden von kommerziellen Züchtern (z. B. Taconic oder Jackson Laboratory) bezogen. Die Tiere werden unter sterilen Bedingungen (einschließlich Streu und Futter) in Isolatoren gehalten.

Eine definierte Anzahl von Zellen (z. B. 5 x $10^6$ T-Zellen (z. B. C8166)) wird mit einer geeigneten m.o.i. (z.B. 0.01 $TCID^{50}$) mit HIV infiziert. Die infizierten Zellen werden in Kollagenschwämme eingebracht. Die so vorbehandelten Schwämme werden den Mäusen unter die Rückenhaut implantiert. Die Mäuse werden einmal oder mehrfach täglich peroral, intraperitoneal, subcutan oder intravenös behandelt, wobei die erste Behandlung vor der Implantation liegen kann. Die Behandlungsgruppen umfassen in der Regel 10 Mäuse. Mindestens eine Gruppe wird mit Placebo behandelt, mindestens eine Gruppe mit einer bekanntermaßen wirksamen Substanz (= Positivkontrolle) und in der Regel mehrere Gruppen mit der erfindungsgemäßen Substanz. Die Tagesdosis der erfindungsgemäßen Substanz liegt zwischen 0.01 mg und 100 mg pro kg Körpergewicht. Die Formulierung der Substanzen erfolgt in 2% DMSO/0.5% Methylcellulose in PBS oder einer anderen geeigneten Mischung, die die Löslichkeit der Substanzen unterstützt. Die Behandlungsdauer beträgt in der Regel viereinhalb Tage. Nach der letzten Substanzapplikation werden die Tiere getötet und die Schwämme entnommen. Die virusinfizierten Zellen werden durch Kollagenaseverdau aus dem Schwamm gewonnen.

Aus den Zellen wird Gesamt-RNA gewonnen, die in der quantitativen PCR auf den Gehalt an Virus-RNA überprüft wird. Die Menge an Virus-RNA wird anhand der Menge eines house-keeping Gens (z. B. GAPDH) normalisiert. Ermittelt wird die Menge an HIV-RNA nach Substanzbehandlung im Vergleich zur placebobehandelten Kontrollgruppe. Wurde ein HIV verwendet, das eine Luziferase trägt, kann zusätzlich oder ersatzweise eine Luziferase-Messung durchgeführt werden. In diesem Fall wird die HIV-Menge über die Höhe des Luziferase-Signals bestimmt, da es in diesem Fall als Maß für die Virusreplikation dient. Die statistische Auswertung erfolgt mittels geeigneter Computerprogramme, z. B. Graph Pad Prism.

## B) Bewertung der pharmakokinetischen Eigenschaften

### *In vivo* Studien

Zur Bestimmung der *in vivo* Pharmakokinetik werden die Testsubstanzen Mäusen, Ratten und Hunden intravenös und oral appliziert. Bei intravenösen Studien wird zur Bestimmung der pharmakokinetischen Eigenschaften der Testsubstanzen eine Dosis von 0.5 mg/kg in allen Spezies gewählt. Bei oraler Gabe wird den Nagern 3 mg/kg appliziert, Hunden 1 mg/kg. Die Testsubstanzen werden zur intravenösen Gabe für Nager in 99% Plasma, 1% DMSO formuliert, bei oraler Gabe in PEG 400, Ethanol und Wasser in variierenden Anteilen. Letzteres Vehikel wird bei Hunden für beide Applikationsrouten verwendet.

Männliche Wistar Ratten werden vor Applikation der Testsubstanzen katheterisiert, so dass die Blutproben mit Hilfe des gelegten Katheters oder durch Punktion der Vena cava zu verschiedenen Zeitenpunkten über ein Intervall von 2 min bis zu 26 h entnommen werden können.

Weiblichen BalbC Mäusen werden die Testsubstanzen als bolus Injektion intravenös appliziert, die Probengewinnung erfolgt in diesem Falle ausschließlich durch Punktion der Vena cava über ein Intervall von 2 min bis zu 26 h. Bei weiblichen Beagle Hunden erfolgt die Applikation ausschließlich durch eine 15 minütige intravenöse Infusion. Die Proben werden durch Punktion der Vena brachialis oder der Vena jugularis über ein Intervall von 10 min bis zu 26 h gewonnen.

Die quantitative Bestimmung der Substanzen erfolgt aus dem gewonnenen Tierplasma und Eichproben, die in Plasma eingestellt werden. Die Plasmaproteine werden durch Fällung mit Acetonitril (ACN) entfernt. Anschließend werden die Proben mittels HPLC auf einer Agilent 1100 LC Anlage (Agilent, Santa Clara, Californien, USA) unter Verwendung unterschiedlicher Säulen, z.B. Luna C8, LichroCart Purospher Star RP18e aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Einsatz eines internen Standards und Verwendung eines validierten Kinetikauswerteprogramms.

Neben Studien zur Bestimmung der pharmakokinetischen Parameter der Testsubstanzen *in vivo* werden Bestimmungen der relativen Bioverfügbarkeit aus Suspension (Formulierung: Tylose Suspension) versus Lösung in der Ratte sowie Hochdosisstudien im Vorfeld von Wirkversuchen und toxikologischen Studien in Mäusen, Ratten und Hunden durchgeführt.

### Plasmastabilität

Das verwendete Plasma der unterschiedlichen Spezies (BalbC Maus, Wistar Ratte, Beagle Hund und Mensch) wird durch Blutabnahme, in mit Li-Heparin beschichtete Monovetten und anschließender Zentrifugation, frisch gewonnen. Zur Bestimmung der Plasmastabilität der Testsubstanzen wird je 500 ng/ml bei 37°C, zu je 2 ml in Plasma inkubiert. Zu verschiedenen Zeitpunkten, über ein Intervall bis zu 3 h, werden Proben dem Inkubationsgefäß entnommen. Die gewonnenen Proben werden mit ACN gefällt, um die Umsetzung zu stoppen und die Plasmaproteine abzutrennen. Die Proben werden äquivalent zu den *in vivo* Studien analysiert.

### Mikrosomale und Hepatozyten Inkubationen

Inkubationen mit Lebermikrosomen verschiedener Spezies (BalbC Maus, Wistar Ratte, Beagle Hund, Mensch) werden in einem Gesamtvolumen von 1.5 ml bei 37°C auf einem modifizierten Multiprobe II® Robotersytem (Canberra Packard) bzw. janus® Robotersystem (Perkin Elmer) durchgeführt.

Die Inkubationsmischungen enthalten jeweils 0.5 μg/ml Testsubstanz sowie 0.2 - 0.5 mg/ml mikrosomales Protein. Zusätzlich wird 0.05 M Phosphatpuffer (pH = 7.4), 1 mM EDTA, 5 mM Glucose-6-phosphat und 1.5 U/ml Glucose-6-phosphat Dehydroxygenase aus *Leuconostoc Mesenteroides* zugesetzt. Die mikrosomale Inkubationen wird durch Zugabe von $NADP^+$ (Endkonzentration: 1 mM) gestartet.

Zur Bestimmung der metabolischen Stabilität der Testsubstanzen in frisch isolierten und kultivierten Ratten-Hunde-, und Humanhepatozyten werden jeweils 1 Millionen Zellen/ml verwendet. Äquivalent dem mikrosomalen Assay werden den Hepatozyten jeweils 0.5 μg/ml Testsubstanz zugesetzt.

Nach 2, 5, 10, 20, 30, 45 und 60 min, oder nach 2, 10, 20, 30, 50, 70 und 90 min bei stabileren Verbindungen, werden 125 μl aus dem jeweiligen Inkubationsansatz entnommen und mit ACN versetzt, um die enzymatischen Reaktionen zu stoppen. Nach der Zentrifugation werden die Proben mittels LC-MS/MS (API 2000 oder 3000, Applied Biosystems) analysiert. "$CL_{blood}$ well-stirred"- und "$F_{max}$ well-stirred"-Werte werden aus den jeweiligen Halbwertszeiten der Verbindungen in den mikrosomalen Inkubationen errechnet. Der Substratabbau kann mit folgenden Formeln beschrieben werden (Houston JB, Utility of in-vitro drug-metabolism data in predicting in-vivo metabolic-clearance, Bioch. Pharm. 47 (9) 1469- 1479 (1994); Obach RS; Baxter JG; Liston TE; Silber BM; Jones BC; MacIntyre F; Rance DJ; Wastall P, The

prediction of human pharmacokinetic parameters from preclinical and in vitro metabolism data, J. Pharmacol. Exp. Ther. 283 (1) 46- 58 (1997)):

$$CL'_{intrinsic}\ [ml/(min \cdot kg)] = (0.693/\text{in vitro } t_{1/2}\ [min])\ (\text{Lebergewicht [g Leber/kg Körpergewicht]})\ (\text{mikrosomales Protein [mg]/Lebergewicht [g]})/(\text{mikrosomales Protein [mg]/Inkubationsvolumen [ml]})$$

Die Blut-Clearance "$CL_{blood}$" wird ohne die Berücksichtigung von Proteinbindungen durch das "well-stirred" Modell beschrieben (Pang KS; Rowland M, Hepatic clearance of drugs. I. Theoretical considerations of a "well-stirred" model and a "parallel tube" model. Influence of hepatic blood flow, plasma and blood cell binding, and the hepatocellular enzymatic activity on hepatic drug clearance, J Pharmacokinet Biopharm 5 (6): 625-53 (1977)):

$$CL_{blood}\ \text{well-stirred}\ [l/(h \cdot kg)] = (Q_H\ [l/(h \cdot kg)] \cdot CL'_{intrinsic}\ [l/(h \cdot kg)])\ /\ (Q_H\ [l/(h \cdot kg)] + CL'_{intrinsic}\ [l/(h \cdot kg)])$$

Für die Ratten beträgt das spezifische Lebergewicht 32 g/kg Körpergewicht und der hepatische Blutfluss 4.2 1/(h·kg). Der spezifische mikrosmale Proteingehalt der Rattenleber wurde mit 40 mg/g Leber veranschlagt. Die spezifischen Hochrechnungsfaktoren weiterer Spezies sind in folgender Tabelle wiedergegeben und beruhen zum Teil auf Literaturdaten, zum Teil auf eigenen Bestimmungen. Für Hepatozyten wird eine Zellzahl von 110 Mio/g Leber bei allen Spezies zur Berechnung herangezogen.

| | Maus m | Maus w | Ratte m | Hund m/w | Mensch m/w |
|---|---|---|---|---|---|
| Mikrosomales Protein/g Leber [mg] | 40 | 40 | 40 | 40 | 40 |
| Leber [g]/kg Körpergewicht | 50 | 43 | 32 | 39 | 21 |
| Leberblutfluss [1/(h·kg)] | 5,4 | 5,4 | 4,2 | 2,1 | 1,32 |

**C) Ausführungsbeispiele für pharmazeutische Zusammensetzung_en**

Die erfindungsgemäßen Verbindungen können folgendermaßen in pharmazeutische Zubereitungen überführt werden:

Tablette:

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

Herstellung:

Die Mischung aus erfindungsgemäßer Verbindung, Lactose und Stärke wird mit einer 5%igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Lösung:**

Zusammensetzung:

500 mg der Verbindung von Beispiel 1, 2.5 g Polysorbat und 97 g Polyethylenglycol 400. Einer Einzeldosis von

100 mg der erfindungsgemäßen Verbindung entsprechen 20 g orale Lösung.

Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

i.v. Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5%, PEG 400 Lösung 30%) gelöst. Die Lösung wird steril filtriert und in sterile und pyrogenfreie Injektionsbehältnisse abgefüllt.

## Patentansprüche

1. Verbindung der Formel

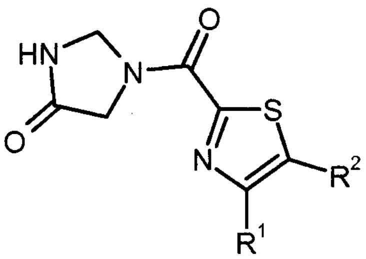

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,
worin
($C_1$-$C_4$)-Alkyl und ($C_1$-$C_4$)-Alkoxy ihrerseits ein- bis dreifach, gleich oder verschieden, mit Resten substituiert sein können, die ausgewählt werden aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, ($C_1$-$C_4$)-Alkyl, Trifluormethyl, Hydroxy, ($C_1$-$C_4$)-Alkoxy, Trifluormethoxy, Oxo, Amino, Mono-($C_1$-$C_1$)-alkylamino und Di-($C_1$-$C_4$)-alkylamino substituiert sein können, und
$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,
worin
($C_1$-$C_4$)-Alkyl und ($C_1$-$C_4$)-Alkoxy ihrerseits ein- bis dreifach, gleich oder verschieden, mit Resten substituiert sein können, die ausgewählt werden aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, ($C_1$-$C_4$)-Alkyl, Trifluormethyl, Hydroxy, ($C_1$-$C_4$)-Alkoxy, Trifluormethoxy, Oxo, Amino, Mono-($C_1$-$C_4$)-alkylamino und Di-($C_1$-$C_4$)-alkylamino substituiert sein können,

oder eines ihrer Salze, ihrer Solvate oder der Solvate ihrer Salze.

2. Verbindung nach Anspruch 1, **dadurch gekennzeichnet, dass**

$R^1$ für Phenyl steht,

wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy, und
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,
worin
($C_1$-$C_4$)-Alkoxy seinerseits ein- bis dreifach, gleich oder verschieden, mit Resten substituiert sein kann, die ausgewählt werden aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4- bis 7-gliedriges Heterocyclyl,
wobei die letztgenannten Cycloalkyl- und Heterocyclyl-Reste ihrerseits jeweils bis zu dreifach, gleich oder verschieden, mit Halogen, Cyano, ($C_1$-$C_4$)-Alkyl, Trifluormethyl, Hydroxy, ($C_1$-$C_4$)-Alkoxy, Trifluormethoxy, Oxo, Amino, Mono-($C_1$-$C_4$)-alkylamino und Di-($C_1$-$C_4$)-alkylamino substituiert sein können,

oder eines ihrer Salze, ihrer Solvate oder der Solvate ihrer Salze.

**3.** Verbindung nach Anspruch 1 oder 2, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Methyl und Methoxy, und
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl, Trifluormethoxy, Methyl und ($C_1$-$C_3$)-Alkoxy,

oder eines ihrer Salze, ihrer Solvate oder der Solvate ihrer Salze.

**4.** Verbindung nach einem der Ansprüche 1 bis 3, **dadurch gekennzeichnet, dass** sie der Formel

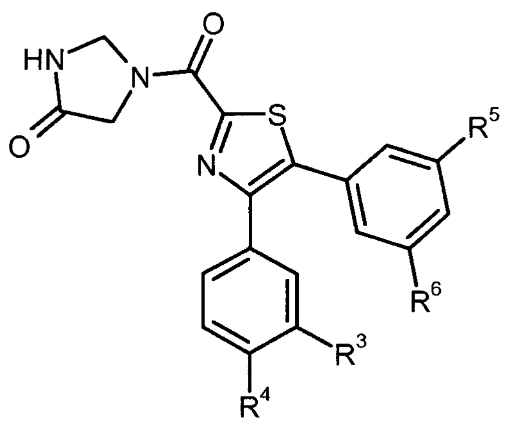

(Ia),

entspricht, in welcher

$R^3$ für Halogen oder Cyano steht,
$R^4$ für Wasserstoff oder Halogen steht,
$R^5$ für Halogen, Cyano oder Trifluormethyl steht, und
$R^6$ für Wasserstoff oder Halogen steht,

oder eines ihrer Salze, ihrer Solvate oder der Solvate ihrer Salze.

**5.** Verbindung nach Anspruch 4, **dadurch gekennzeichnet, dass**

$R^3$ für Fluor, Chlor oder Cyano steht,

$R^4$ für Wasserstoff, Chlor oder Fluor steht,
$R^5$ für Fluor, Chlor oder Cyano steht, und
$R^6$ für Wasserstoff, Chlor oder Fluor steht,

oder eines ihrer Salze, ihrer Solvate oder der Solvate ihrer Salze.

**6.** Verbindung nach Anspruch 4, **dadurch gekennzeichnet, dass**

$R^3$ für Chlor oder Cyano steht,
$R^4$ für Wasserstoff oder Fluor steht,
$R^5$ für Halogen oder Cyano steht, und
$R^6$ für Wasserstoff oder Fluor steht,

oder eines ihrer Salze, ihrer Solvate oder der Solvate ihrer Salze.

**7.** Verfahren zur Herstellung einer Verbindung der Formel (I) nach Anspruch 1, **dadurch gekennzeichnet, dass** eine Verbindung der Formel

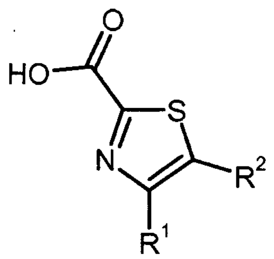

(II),

in welcher
$R^1$ und $R^2$ die in Anspruch 1 angegebene Bedeutung aufweisen,
mit Imidazolidin-4-on oder einem Salz von Imidazolidin-4-on umgesetzt wird.

**8.** Verfahren zur Herstellung einer Verbindung der Formel (I) nach Anspruch 1, **dadurch gekennzeichnet, dass** eine Verbindung der Formel

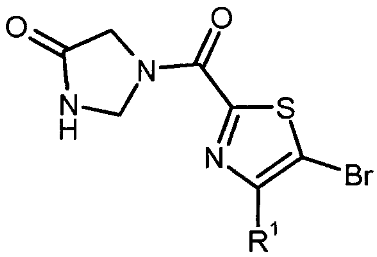

(VII),

in welcher $R^1$ die in Anspruch 1 angegebene Bedeutung aufweist,
unter Suzuki-Kupplungsbedingungen mit einer Verbindung der Formel

$R^2$-Q (IV),

in welcher
$R^2$ die in Anspruch 1 angegebene Bedeutung aufweist und

Q für $-B(OH)_2$, einen Boronsäure-Ester, bevorzugt Boronsäurepinakolester, oder $-BF_3^-K^+$ steht,

umgesetzt wird.

**9.** Verbindung nach einem der Ansprüche 1 bis 6 zur behandlung und/oder Prophylaxe von Krankheiten.

**10.** Verwendung einer Verbindung nach einem der Ansprüche 1 bis 6 zur Herstellung eines Arzneimittels zur Behandlung und/oder Prophylaxe von Krankheiten.

**11.** Verwendung einer Verbindung nach einem der Ansprüche 1 bis 6 zur Herstellung eines Arzneimittels zur Behandlung und/oder Prophylaxe von retroviralen Erkrankungen.

**12.** Verwendung nach Anspruch 11, **dadurch gekennzeichnet, dass** die retrovirale Erkrankung eine Infektion mit dem HI-Virus ist.

**13.** Arzneimittel enthaltend mindestens eine Verbindung nach einem der Ansprüche 1 bis 6 in Kombination mit mindestens einem weiteren Wirkstoff.

**14.** Arzneimittel enthaltend mindestens eine Verbindung nach einem der Ansprüche 1 bis 6 in Kombination mit einem inerten nicht-toxischen pharmazeutisch geeigneten Hilfsstoff.

**15.** Arzneimittel nach Anspruch 13 oder 14 zur Behandlung und/oder Prophylaxe von retroviralen Erkrankungen.

**16.** Arzneimittel nach Anspruch 15, **dadurch gekennzeichnet, dass** die retrovirale Erkrankung eine Infektion mit dem HI-Virus ist.

**17.** Verbindung nach einem der Ansprüche 1 bis 6 oder Arzneimittel nach einem der Ansprüche 13 bis 16 zur Verwendung in einem Verfahren zur Bekämpfung von viralen Erkrankungen in Menschen und Tieren.

## Claims

**1.** A compound of the formula

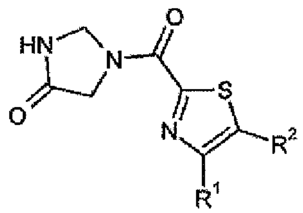 (I),

in which

$R^1$ represents phenyl,
whereby phenyl is substituted with 1 to 3 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkyl and $(C_1-C_4)$-alkoxy in turn maybe substituted one to three times identically or differently with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
whereby the last-mentioned cycloalkyl and heterocyclyl residues in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylamino and di-$(C_1-C_4)$-alkylamino, and
$R^2$ represents phenyl,
whereby phenyl is substituted with 1 to 3 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkyl and $(C_1-C_4)$-alkoxy in turn maybe substituted one to three times identically or differently with residues selected from the group consisting of halogen, cyano, hydroxy, $(C_1-C_4)$-alkoxy, amino, mono-$(C_1-C_4)$-alkylamino, di-$(C_1C_4)$-alkylamino, $(C_3-C_7)$-cycloalkyl and 4- to 7-membered heterocyclyl,

whereby the last-mentioned cycloalkyl and heterocyclyl residues in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylammo and di-$(C_1-C_4)$-alkylamino,

or one of their salts, their solvates or the solvates of their salts.

**2.** The compound according to Claim 1, **characterized in that**

$R^1$ represents phenyl,
whereby phenyl is substituted with 1 to 2 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy, and
$R^2$ represents phenyl,
whereby phenyl is substituted with 1 to 2 substituents, whereby the substituents are selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
wherein
$(C_1-C_4)$-alkoxy in turn may be substituted one or three times identically or differently with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
whereby the last-mentioned cycloalkyl and heterocyclyl residues in turn may each be substituted up to three times identically or differently with halogen, cyano, $(C_1-C_4)$-alkyl, trifluoromethyl, hydroxy, $(C_1-C_4)$-alkoxy, trifluoromethoxy, oxo, amino, mono-$(C_1-C_4)$-alkylamino and di-$(C_1-C_4)$-alkylamino,

or one of their salts, their solvates or the solvates of their salts

**3.** The compound according to Claim 1 or 2, **characterized in that**

$R^1$ represents phenyl,
whereby phenyl is substituted with 1 to 2 substituents, whereby the substituents are selected independently of one another from the group consisting of halogen, cyano, trifluoromethyl, methyl and methoxy, and
$R^2$ represents phenyl,
whereby phenyl is substituted with 1 to 2 substituents, where the substituents are selected independently of one another from the group consisting of halogen, cyano, trifluoromethyl, trifluoromethoxy, methyl and $(C_1-C_3)$-alkoxy,

or one of their salts, their solvates or the solvates of their salts

**4.** The compound according to any of Claims 1 to 3, **characterized in that** it corresponds to the formula

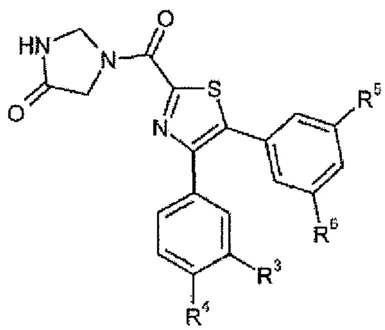

(Ia),

in which

$R^3$ represents halogen or cyano,
$R^4$ represents hydrogen or halogen,
$R^5$ represents halogen, cyano or trifluoromethyl, and
$R^6$ represents hydrogen or halogen,

or one of their salts, their solvates or the solvates of their salts

5. The compound according to Claim 4, **characterized in that**

$R^3$ represents fluorine, chlorine or cyano,
$R^4$ represents hydrogen, chlorine or fluorine,
$R^5$ represents fluorine, chlorine or cyano, and
$R^6$ represents hydrogen, chlorine or fluorine,

or one of their salts, their solvates or the solvates of their salts

6. The compound according to Claim 4, **characterized in that**

$R^3$ represents chlorine or cyano,
$R^4$ represents hydrogen or fluorine,
$R^5$ represents halogen or cyano, and
$R^6$ represents hydrogen or fluorine,

or one of their salts, their solvates or the solvates of their salts

7. A method for preparing a compound of Formula (I) according to Claim 1, **characterized in that** a compound of the formula

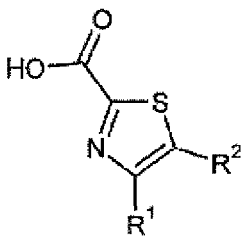 (II),

in which
$R^1$ and $R^2$ have the meaning given in Claim 1,
is reacted with imidazolidin-4-one or a salt of imidazolidin-4-one.

8. The method for preparing a compound of Formula (I) according to Claim 1, **characterized in that** a compound of the formula

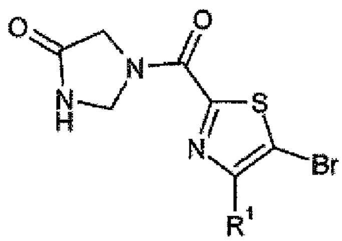 (VII),

in which
$R^1$ has the meaning given in Claim 1,
is reacted under Suzuki coupling conditions with a compound of the formula

$R^2$-Q (IV),

in which
$R^2$ has the meaning given in Claim 1 and

Q represents $-B(OH)_2$, a boronic acid ester, preferably a boronic acid pinacol ester, or $-BF_3^-K^+$.

**9.** The compound according to any of Claims 1 to 6 for the treatment and/or prophylaxis of diseases.

**10.** Use of the compound according to any of Claims 1 to 6 in the manufacture of a medicament for the treatment and/or prophylaxis of diseases.

**11.** Use of the compound according to any of Claims 1 to 6 in the manufacture of a medicament for the treatment and/or prophylaxis of retroviral diseases.

**12.** Use according to Claim 11, **characterized in that** the retroviral disease is an HIV infection.

**13.** Medicament containing at least one compound according to any of Claims 1 to 6 in combination with at least one additional active ingredient.

**14.** Medicament containing at least one compound according to any of Claims 1 to 6 in combination with at least one inert, non-toxic, pharmaceutically appropriate excipient.

**15.** Medicament according to Claim 13 or 14 for the treatment and/or prophylaxis of retroviral diseases.

**16.** Medicament according to Claim 15, **characterized in that** the retroviral disease is an HIV infection.

**17.** The compound according to any of Claims 1 to 6 or a medicament according to any of Claims 13 to 16 for use in a method to combat viral diseases in humans and animals.

## Revendications

**1.** Un composé de la formule

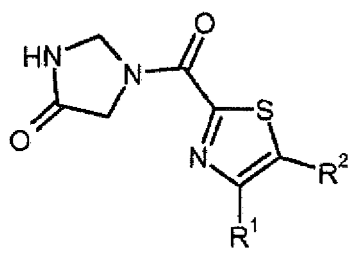

(I),

dans laquelle

$R^1$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, $(C_1\text{-}C_4)$-alkyl et $(C_1\text{-}C_4)$-alkoxy,
dans laquelle
$(C_1\text{-}C_4)$-alkyle et $(C_1\text{-}C_4)$-alkoxy peuvent à leur tour être substitués une à trois fois, de façon égale ou différente, par des résidus sélectionnés parmi le groupe constitué par halogène, cyano, hydroxy, $(C_1\text{-}C_4)$-alkoxy, amino, mono-$(C_1\text{-}C_4)$-alkylamino, di-$(C_1\text{-}C_4)$-alkylamino, $(C_3\text{-}C_7)$-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, $(C_1\text{-}C_4)$-alkyle, trifluorométhyle, hydroxy, $(C_1\text{-}C_4)$-alkoxy, trifluorométhoxy, oxo, amino, mono-$(C_1\text{-}C_4)$-alkylamino et di-$(C_1\text{-}C_4)$-alkylamino, et
$R^2$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, $(C_1\text{-}C_4)$-alkyl et $(C_1\text{-}C_4)$-alkoxy,
dans laquelle
$(C_1\text{-}C_4)$-alkyle et $(C_1\text{-}C_4)$-alkoxy peuvent à leur tour être substitués une à trois fois, de façon égale ou différente, par des résidus sélectionnés parmi le groupe constitué par halogène, cyano, hydroxy, $(C_1\text{-}C_4)$-alkoxy, amino,

mono-($C_1$-$C_4$)-alkylamino, di-($C_1$-$C_4$)-alkylarnino, ($C_3$-$C_7$)-cycloalkyle et hétérocycle ayant 4 à 7 membres, dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyle, hydroxy, ($C_1$-$C_4$)-alkoxy, trifluorométhoxy, oxo, amino, mono-($C_1$-$C_4$)-alkylamino et di-($C_1$-$C_4$)-alkylamino,

ou un de leurs sels, solvats ou les solvats de leurs sels.

**2.** Le composé selon la revendication 1, **caractérisé en ce que**

$R^1$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy, et
$R^2$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy,
dans laquelle
($C_1$-$C_4$)-alkoxy peut à son tour être substitué une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyle, hydroxy, ($C_1$-$C_4$)-alkoxy, trifluorométhoxy, oxo, amino, mono-($C_1$-$C_4$)-alkylamino et di-($C_1$-$C_4$)-alkylamino,

ou un de leurs sels, solvats ou les solvats de leurs sels.

**3.** Le composé selon la revendication 1 ou 2, **caractérisé en ce que**

$R^1$ représente phényle,
où phényle est substitué par 1 ou 2 substituants, les substituants étant sélectionnés indépendamment l'un de l'autre parmi le groupe constitué par halogène, cyano, trifluorométhyle, methyle et méthoxy,et
$R^2$ représente phényle,
où phényle est substitué par 1 ou 2 substituants, les substituants étant sélectionnés indépendamment l'un de l'autre parmi le groupe constitué par halogène, cyano, trifluorométhyle, trifluorométhoxy, methyle et ($C_1$-$C_3$)-alkoxy,

ou un de leurs sels, solvats ou les solvats de leurs sels.

**4.** Le composé selon l'une quelconque des revendications 1 à 3, **caractérisé en ce qu'**il correspond à la formule

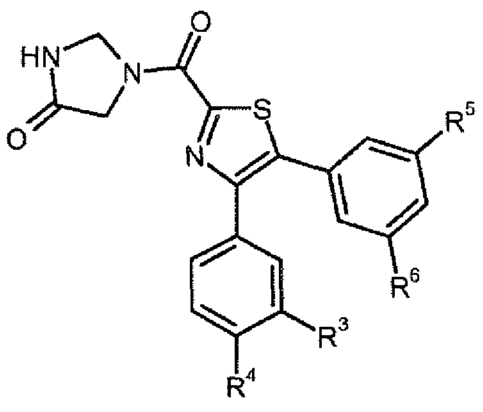

(Ia),

dans laquelle

$R^3$ représente halogène ou cyano,
$R^4$ représente hydrogène ou halogène,
$R^5$ représente halogène, cyano ou trifluorométhyle, et
$R^6$ représente hydrogène ou halogène,

ou un de leurs sels, solvats ou les solvats de leurs sels.

5. Le composé selon la revendication 4, **caractérisé en ce que**

$R^3$ représente fluor, chlore ou cyano,
$R^4$ représente hydrogène, chlore ou fluor,
$R^5$ représente fluor, chlore ou cyano, et
$R^6$ représente hydrogène, chlore ou fluor,

ou un de leurs sels, solvats ou les solvats de leurs sels.

6. Le composé selon la revendication 4, **caractérisé en ce que**

$R^3$ représente chlore ou cyano,
$R^4$ représente hydrogène ou fluor,
$R^5$ représente halogène ou cyano, et
$R^6$ représente hydrogène ou fluor,

ou un de leurs sels, solvats ou les solvats de leurs sels.

7. Un procédé de production d'un composé de la formule (I) selon la revendication 1, **caractérisé en ce qu'**un composé de la formule suivante

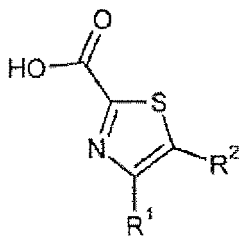 (II),

dans laquelle
$R^1$ et $R^2$ ont la signification spécifiée dans la revendication 1,
est réalisée à l'aide de l'imidazolidine-4 ou d'un sel de l'imidazolidine-4.

8. Le procédé de production d'un composé de la formule (I) selon la revendication 1, **caractérisé en ce qu'**un composé de la formule suivante

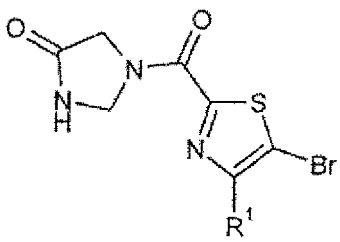 (VII),

dans laquelle
$R^1$ a la signification spécifiée dans la revendication 1,
est réalisée dans des conditions de couplage Suzuki, avec composé de la formule

$R^2$-Q (IV),

dans laquelle
$R^2$ a la signification spécifiée dans la revendication 1 et

Q représente $-B(OH)_2$, un ester d'acide boronique, de préférence un ester pinacolique d'acide boronique

ou $-BF_3^-X^+$.

**9.** Le composé selon l'une quelconque des revendications 1 à 6 destiné au traitement et/ou à la prévention de maladies.

**10.** Utilisation d'un composé selon l'une quelconque des revendications 1 à 6 pour produire un médicament destiné au traitement et/ou la prévention de maladies.

**11.** Utilisation d'un composé selon l'une quelconque des revendications 1 à 6 pour produire un médicament destiné au traitement et/ou à la prévention de maladies rétrovirales.

**12.** Utilisation d'un composé selon la revendication 11, **caractérisée en ce que** la maladie rétrovirale est une infection au VIH.

**13.** Un médicament contenant au moins un composé selon l'une quelconque des revendications 1 à 6 en combinaison avec au moins une substance active supplémentaire.

**14.** Un médicament contenant au moins un composé selon l'une quelconque des revendications 1 à 6 en combinaison avec au moins un excipient inerte, non toxique, pharmaceutiquement acceptable.

**15.** Un médicament selon la revendication 13 ou 14 destiné au traitement et/ou à la prévention de maladies rétrovirales.

**16.** Un médicament selon la revendication 15, **caractérisé en ce que** la maladie rétrovirale est une infection au VIH.

**17.** Le composé selon l'une quelconque des revendications 1 à 6 ou médicament selon l'une quelconque des revendications 13 à 16 destinée à être utilisée dans une méthode de lutter contre les maladies virales chez les humains et les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 377457 A1
- EP 388909 A2
- WO 0378413 A1
- WO 2004058255 A1
- WO 03041711 A1
- WO 2005115389 A2
- WO 2006023462 A1
- WO 2006062984 A
- WO 2006062982 A2
- WO 02055079 A
- GB 453061 A


### In der Beschreibung aufgeführte Nicht-Patentliteratur


- **PALELLA et al.** *N. Engl. J. Med.,* 1998, vol. 238, 853-860
- **FLEXNER.** *Nature Reviews Drug Discovery,* 2007, vol. 6, 959-966
- **CARPENTER et al.** *J. Am. Med. Assoc.,* 2000, vol. 283, 381-390
- **FINZI et al.** *Nature Med.,* 1999, vol. 5, 512-517
- **RAMRATNAM et al.** *Nature Med.,* 2000, vol. 6, 82-85
- **KAVLICK ; MITSUYA.** Antiretroviral Chemotherapy. ASM Press, 2001, 279-312
- **HOUSTON JB.** Utility of in-vitro drug-metabolism data in predicting in-vivo metabolic-clearance. *Bioch. Pharm.,* 1994, vol. 47 (9), 1469-1479
- **OBACH RS ; BAXTER JG ; LISTON TE ; SILBER BM ; JONES BC ; MACINTYRE F ; RANCE DJ ; WASTALL P.** The prediction of human pharmacokinetic parameters from preclinical and in vitro metabolism data. *J. Pharmacol. Exp. Ther,* 1997, vol. 283 (1), 46-58
- **PANG KS ; ROWLAND M.** Hepatic clearance of drugs. I. Theoretical considerations of a ''well-stirred'' model and a ''parallel tube'' model. Influence of hepatic blood flow, plasma and blood cell binding, and the hepatocellular enzymatic activity on hepatic drug clearance. *J Pharmacokinet Biopharm,* 1977, vol. 5 (6), 625-53